# EUROPÄISCHE PATENTSCHRIFT

(11) **EP 2 245 152 B1**
(45) Veröffentlichungstag und Bekanntmachung des Hinweises auf die Patenterteilung: **01.05.2019**
(21) Anmeldenummer: 09712033.1
(22) Anmeldetag: 19.02.2009
(51) Int. Cl.: C12N 9/96, C12N 9/06, C12Q 1/32

(54) **STABILISIERUNG VON DEHYDROGENASEN MIT STABILEN COENZYMEN**
STABILISATION OF DEHYDROGENASES WITH STABLE COENZYMES
STABILISATION DE DÉHYDROGÉNASES À L'AIDE DE COENZYMES STABLES

(30) Priorität: 19.02.2008 EP 08003054
(43) Veröffentlichungstag der Anmeldung: 03.11.2010
(73) Patentinhaber: F. Hoffmann-La Roche AG, 4070 Basel (CH); Roche Diabetes Care GmbH, 68305 Mannheim (DE)
(72) Erfinder: HEINDL, Dieter, 82396 Pähl (DE); HORN, Carina, 68647 Biblis (DE); GAESSLER-DIETSCHE, Claudia, 69198 Schriesheim (DE); HOENES, Joachim, 64673 Zwingenberg (DE)
(74) Vertreter: Weickmann & Weickmann PartmbB
(86) Internationale Anmeldenummer: PCT/EP2009/001206
(87) Internationale Veröffentlichungsnummer: WO 2009/103540

(56) Entgegenhaltungen:
- WO-A-01/49247
- WO-A-98/33936
- WO-A-2007/012494
- WO-A1-2007/012494
- US-A1- 2007 026 476
- LIAO MIN-HUNG ET AL: "Characteristics of magnetic nanoparticles-bound YADH in water/AOT/isooctane microemulsions" JOURNAL OF MOLECULAR CATALYSIS B ENZYMATIC, Bd. 18, Nr. 1-3, 13. September 2002 (2002-09-13), Seiten 81-87, XP002502967 ISSN: 1381-1177
- SLAMA J T ET AL: "INHIBITION OF NAD GLYCOHYDROLASE AND ADP-RIBOSYLTRANSFERASES BY CARBOCYCLIC ANALOGUES OF OXIDIZED NAD" BIOCHEMISTRY, Bd. 28, Nr. 19, 1989, Seiten 7688-7694, XP002502968 ISSN: 0006-2960 in der Anmeldung erwähnt

## Beschreibung

Die vorliegende Erfindung betrifft ein Verfahren zur Stabilisierung eines Enzyms durch Lagerung des Enzyms in Gegenwart eines stabilen Coenzyms. Weiterhin betrifft die vorliegende Erfindung ein mit einem stabilen Coenzym stabilisiertes Enzym sowie dessen Verwendung in Testelementen zum Nachweis von Analyten.

Biochemische Messsysteme sind wichtige Bestandteile klinisch relevanter Analyseverfahren. Hierbei steht die Messung von Analyten, z.B. Metaboliten oder Substraten, im Vordergrund, welche mit Hilfe eines Enzyms direkt oder indirekt bestimmt werden. Die Analyten werden hierbei mit Hilfe eines Enzym-Coenzym-Komplexes umgesetzt und anschließend quantifiziert. Dabei wird der zu bestimmende Analyt mit einem geeigneten Enzym und einem Coenzym in Kontakt gebracht, wobei das Enzym meist in katalytischen Mengen eingesetzt wird. Das Coenzym wird durch die enzymatische Reaktion verändert, z.B. oxidiert bzw. reduziert. Dieser Vorgang kann direkt oder durch einen Mediator elektrochemisch oder photometrisch erfasst werden. Eine Kalibrierung liefert einen direkten Zusammenhang des Messwerts mit der Konzentration des zu bestimmenden Analyten.

Coenzyme sind organische Moleküle, die kovalent oder nicht-kovalent an ein Enzym gebunden sind und durch die Umsetzung des Analyten verändert werden. Prominente Beispiele für Coenzyme sind Nicotinamid-Adenin-Dinukleotid (NAD) bzw. Nicotinamid-Adenin-Dinukleotidphosphat (NADP), aus denen durch Reduktion NADH bzw. NADPH entstehen.

Aus dem Stand der Technik bekannte Messsysteme zeichnen sich durch eine zeitlich begrenzte Haltbarkeit sowie durch spezielle Anforderungen an die Umgebung, wie Kühlung oder trockene Lagerung, zur Erzielung dieser Haltbarkeit aus. Bei bestimmten Anwendungsformen, z.B. bei Tests, die vom Endverbraucher selbst durchgeführt werden, wie etwa beim Blutglucose-Selbstmonitoring, können daher Fehlergebnisse durch falsche, unbemerkte Fehllagerung vorkommen. Besonders die Erschöpfung von Trocknungsmitteln durch zu langes Öffnen der Primärverpackungen kann zu Fehlmessungen führen, die bei manchen Systemen vom Verbraucher kaum zu erkennen sind.

Eine bekannte Maßnahme, die zur Erhöhung der Stabilität von biochemischen Messsystemen eingesetzt wird, ist die Verwendung stabiler Enzyme, z.B. die Verwendung von Enzymen aus thermophilen Organismen. Weiterhin besteht die Möglichkeit, Enzyme durch chemische Modifizierung, z.B. Quervernetzung, oder durch Mutagenese zu stabilisieren. Darüber hinaus können auch Enzymstabilisatoren, wie z.B. Trehalose, Polyvinylpyrrolidon und Serumalbumin, zugesetzt werden oder die Enzyme z.B. durch Photopolymerisation in Polymernetzwerke eingeschlossen werden.

Weiterhin wird versucht, die Haltbarkeit biochemischer Messsysteme durch Verwendung stabiler Mediatoren zu verbessern. So wird durch die Verwendung von Mediatoren mit möglichst niedrigem Redoxpotential die Spezifität von Tests erhöht und Störungen während der Reaktion eliminiert. Eine untere Grenze für das Redoxpotential von Mediatoren bilden jedoch die Redoxpotentiale der Enzym/Coenzymkomplexe. Werden diese unterschritten, wird die Reaktion mit den Mediatoren verlangsamt oder gar unterbunden.

Alternativ können auch biochemische Messsysteme ohne Mediatoren verwendet werden, bei denen beispielsweise eine direkte Detektion von Coenzymen, z.B. des Coenzyms NADH, erfolgt. Ein Nachteil solcher Messsysteme besteht jedoch darin, dass Coenzyme, wie NAD und NADP, instabil sind.

NAD und NADP sind basenlabile Moleküle, deren Abbauwege in der Literatur beschrieben sind (N.J. Oppenheimer in The Pyridine Nucleotide Coenzyms Academic Press New York, London 1982, Hrsg. J. Everese, B. Anderson, K. You, Kapitel 3, Seiten 56-65). Im wesentlichen entsteht beim Abbau von NAD bzw. NADP ADP-Ribose, indem die Glycosyl-Bindungen zwischen der Ribose und der Pyridineinheit gespalten wird. Die reduzierten Formen NADH und NADPH sind hingegen säurelabil: z.B. ist die Epimerisierung ein bekannter Abbauweg. In beiden Fällen liegt der Instabilität von NAD/NADP und NADH/NADPH die Labilität der Glykosyl-Bindung zwischen der Ribose- und der Pyridineinheit zugrunde. Aber auch unter nicht drastischen Bedingungen, wie z.B. in wässriger Lösung, geschieht die Hydrolyse der Coenzyme NAD bzw. NADP allein schon durch die Umgebungsfeuchte. Diese Instabilität kann zu Ungenauigkeiten bei der Messung von Analyten führen.

Eine Reihe von NAD/NADP-Derivaten wird z.B. in B.M. Anderson in the Pyridine Nucleotide Coenzymes, Academic Press New York, London 1982 , Hrsg. J. Everese, B. Anderson, K. You, Kapitel 4 beschrieben. Die meisten dieser Derivate werden allerdings nicht gut von Enzymen akzeptiert. Das einzige Derivat, das daher bisher für diagnostische Tests verwendet wurde, ist 3-Acetylpyridin-Adenin-Dinukleotid (Acetyl NAD), welches erstmals 1956 beschrieben wurde (N.O. Kaplan, J. Biol. Chem. (1956), 221, 823). Auch dieses Coenzym zeigt eine geringe Akzeptanz von Enzymen und eine Änderung im Redoxpotential.

In WO 01/94370 ist die Verwendung weiterer NAD-Derivate mit einer modifizierten Pyridin-Gruppe beschrieben. Modifikationen der Nicotinamid-Gruppe haben jedoch generell direkten Einfluss auf die katalytische Reaktion. In den meisten Fällen ist dieser Einfluss negativ.

In einem weiteren Stabilisierungskonzept wurde die Ribose-Einheit verändert, um dadurch die Stabilität der Glycosyl-Bindung zu beeinflussen. Dieses Vorgehen interferiert nicht direkt mit der katalytischen Reaktion der Nicotinamid-Gruppe. Es kann jedoch ein indirekter Einfluss bestehen, sobald das Enzym eine starke und spezifische Bindung an die Ribose-Einheit aufweist. Kaufmann et al. offenbaren diesbezüglich in WO 98/33936 und US 5,801,006 bzw. in WO 01/49247 eine Reihe von Thioribose-NAD Derivaten. Ein Zusammenhang zwischen der Modifizierung der Nicotinamid-Riboseeinheit und der Aktivität der Derivate in enzymatischen Reaktionen wurde bisher jedoch nicht gezeigt.

carbaNAD, ein Derivat ohne Glycosyl-Bindung, wurde erstmals 1988 beschrieben (J.T. Slama, Biochemistry 1989, 27, 183 und Biochemistry 1989, 28, 7688). Die Ribose wird hierin durch eine carbazyklische Zucker-Einheit substituiert. carbaNAD wurde zwar als Substrat für Dehydrogenasen beschrieben, seine Aktivität wurde bisher jedoch nicht in biochemischen Nachweisverfahren in der Klinik nachgewiesen.

Ein ähnlicher Ansatz wurde später von G.M. Blackburn, Chem. Comm., 1996, 2765 beschrieben, um carbaNAD mit einer Methylenbisphosphonat-Verbindung an Stelle des natürlichen Pyrophosphats herzustellen. Das Methylenbisphosphonat zeigt erhöhte Stabilität gegen Phosphatasen und wurde als Inhibitor für ADP-Ribosylcyclase verwendet. Eine Stabilitätserhöhung gegenüber Hydrolyse war nicht das Ziel (J.T. Slama, G.M. Blackburn).

WO 2007/012494 und US 11/460,366 offenbaren stabile NAD/NADH bzw. NADP/NADPH Derivate, Enzym-Komplexe dieser Derivate und ihre Verwendung in biochemischen Nachweisverfahren und Reagenzienkits.

Die der vorliegenden Erfindung zugrundeliegende Aufgabe war es, Verfahren zur Stabilisierung von Enzymen, insbesondere zur Langzeitstabiliserung von Enzymen bereitzustellen.

Diese Aufgabe wird gelöst durch ein Verfahren zur Stabilisierung eines Enzyms gemäß Anspruch 1, wobei man das Enzym in Gegenwart eines stabilen Coenzyms lagert. Überraschenderweise wurde gefunden, dass mit Hilfe eines stabilen Coenzyms wie in Anspruch 1 definiert eine Langzeitstabiliserung von mehreren Wochen bzw. Monaten bei hoher relativer Feuchte oder sogar in flüssiger Phase und bei erhöhten Temperaturen möglich ist. Diese Erkenntnis ist überraschend, da bekannt ist, dass Enzyme in Gegenwart von nativem Coenzym zwar eine erhöhte Kurzzeitstabilität für einige Stunden besitzen (Bertoldi et al., Biochem. J. 389, (2005), 885-898; van den Heuvel et al. (J. Biol. Chem. 280 (2005), 32115-32121; und Pan et al. (J. Chin. Biochem. Soc. Vol. 3 (1974), pp. 1-8), aber eine geringere Haltbarkeit über einen längeren Zeitraum aufweisen (Nutrition Reviews 36 (1978), 251-254).

Gegenüber diesen Erkenntnissen gegenüber dem Stand der Technik war es überraschend, dass ein Enzym in Gegenwart eines stabilen Coenzyms eine deutlich erhöhte Langzeitstabilität als ein Enzym in Gegenwart eines nativen Coenzyms besitzt, insbesondere da die stabilen Coenzyme eine niedrigere Bindungskonstante mit dem Enzym als das native Coenzym besitzen.

Das durch das erfindungsgemäße Verfahren stabilisierte Enzym ist ein Coenyzm-abhängiges Enzym. Geeignete Enzyme sind z.B. Dehydrogenasen, ausgewählt aus einer Glucose-Dehydrogenase (E.C.1.1.1.47), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), alpha-Hydroxybutyrat-Dehydrogenase, Sorbitol-Dehydrogenase oder Aminosäure-Dehydrogenase, z. B. L-Aminosäure-Dehydrogenase (E.C.1.4.1.5). Weitere geeignete Enzyme sind Oxidasen, wie etwa Glucoseoxidase (E.C.1.1.3.4) oder Cholesterinoxidase (E.C.1.1.3.6) bzw. Aminotransferasen, wie z.B. Aspartat oder Alanin Aminotransferase, 5'-Nukleotidase oder Creatin-Kinase. Vorzugsweise ist das Enzym Glucose-Dehydrogenase.

Als besonders bevorzugt hat sich im Rahmen des erfindungsgemäßen Verfahrens der Einsatz einer mutierten Glucose-Dehydrogenase erwiesen. Der Begriff "Mutante", wie er im Rahmen der vorliegenden Anmeldung verwendet wird, bezeichnet eine genetisch veränderte Variante eines nativen Enzyms, welche bei gleicher Anzahl an Aminosäuren eine gegenüber dem Wildtyp-Enzym veränderte Aminosäuresequenz besitzt, d.h. sich in mindestens einer Aminosäure vom Wildtyp-Enzym unterscheidet. Die Einführung der Mutation(en) kann ortsspezifisch oder nicht-ortsspezifisch, bevorzugt ortsspezifisch unter Verwendung von im Fachbereich bekannten rekombinanten Methoden, erfolgen, wobei entsprechend den jeweiligen Anforderungen und Bedingungen mindestens ein Aminosäureaustausch innerhalb der Aminosäuresequenz des nativen Enzyms resultiert. Besonders bevorzugt weist die Mutante eine gegenüber dem Wildtyp-Enzym erhöhte thermische oder hydrolytische Stabilität auf.

Die mutierte Glucose-Dehydrogenase kann die gegenüber der korrespondierenden Wildtyp-Glucose-Dehydrogenase veränderte(n) Aminosäure(n) grundsätzlich an einer beliebigen Position ihrer Aminosäuresequenz enthalten. Vorzugsweise umfasst die mutierte Glucose-Dehydrogenase eine Mutation an mindestens einer der Positionen 96, 170 und 252 der Aminosäuresequenz der Wildtyp-Glucose-Dehydrogenase, wobei Mutanten mit Mutationen an Position 96 und Position 170 bzw. Mutationen an Position 170 und Position 252 besonders bevorzugt sind. Als vorteilhaft hat es sich erwiesen, wenn die mutierte Glucose-Dehydrogenase neben diesen Mutationen keine weiteren Mutationen enthält.

Die Mutation an den Positionen 96, 170 und 252 kann grundsätzlich einen beliebigen Aminosäureaustausch umfassen, welcher zu einer Stabilisierung, z.B. einer Erhöhung der thermischen oder hydrolytischen Stabilität, des Wildtyp-Enzyms führt. Vorzugsweise umfasst die Mutation an Position 96 einen Aminosäureaustausch von Glutaminsäure gegen Glycin, während in Bezug auf Position 170 ein Aminosäureaustausch von Glutaminsäure gegen Arginin oder Lysin, insbesondere ein Aminosäureaustausch von Glutaminsäure gegen Lysin, bevorzugt ist. Was die Mutation an Position 252 anbetrifft, so umfasst diese bevorzugt einen Aminosäureaustausch von Lysin gegen Leucin.

Die mutierte Glucose-Dehydrogenase kann durch Mutation einer aus einer beliebigen biologischen Quelle stammenden Wildtyp-Glucose-Dehydrogenase erhalten werden, wobei der Begriff "biologische Quelle" im Sinne dieser Erfindung sowohl Prokaryoten, wie beispielsweise Bakterien, als auch Eukaryoten, wie beispielsweise Säuger und andere Tiere, umfasst. Vorzugsweise entstammt die Wildtyp-Glucose-Dehydrogenase einem Bakterium, wobei es sich besonders bevorzugt um eine Glucose-Dehydrogenase aus Bacillus megaterium, Bacillus subtilis oder Bacillus thuringiensis, insbesondere aus Bacillus subtilis, handelt.

In einer besonders bevorzugten Ausführungsform der vorliegenden Erfindung handelt es sich bei der mutierten Glucose-Dehydrogenase um eine durch Mutation von Wildtyp-Glucose-Dehydrogenase aus Bacillus subtilis erhaltene Glucose-Dehydrogenase, welche die in SEQ ID NO: 1 (GlucDH_E96G_E170K) oder die in SEQ ID NO: 2 (GlucDH_E170K_K252L) dargestellte Aminosäuresequenz besitzt.

Das stabile Coenzym ist ein gegenüber dem nativen Coenzym chemisch verändertes Coenzym der Formel (I) oder (II), welches eine im Vergleich zum nativen Coenyzm höhere Stabilität, (z.B. hydrolytische Stabilität) aufweist. Vorzugsweise ist das stabile Coenyzm unter Testbedingungen gegenüber Hydrolyse stabil. Im Vergleich zum nativen Coenzym kann das stabile Coenzym eine verringerte Bindungskonstante für das Enzym aufweisen, beispielsweise eine um den Faktor von 2 oder mehr verringerte Bindungskonstante.

Ein stabiles Coenzym im Sinne der vorliegenden Erfindung ist die Verbindung der Formel (I)

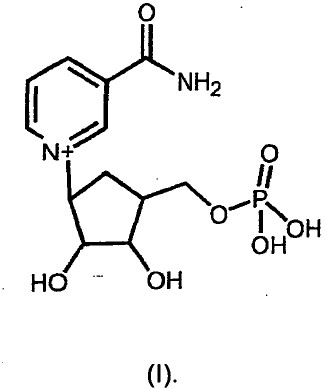

Bevorzugte stabile Derivate von NAD/NADH bzw. NADP/NADPH sind in den zuvor genannten Referenzen beschrieben, auf deren Offenbarung hiermit ausdrücklich Bezug genommen wird. Besonders bevorzugte stabilisierte Coenzyme sind in WO 2007/012494 bzw. US 11/460,366 beschrieben, auf deren Offenbarung hiermit ausdrücklich Bezug genommen wird. Das stabile Coenzym wird besonders bevorzugt aus Verbindungen mit der allgemeinen Formel (II) ausgewählt: 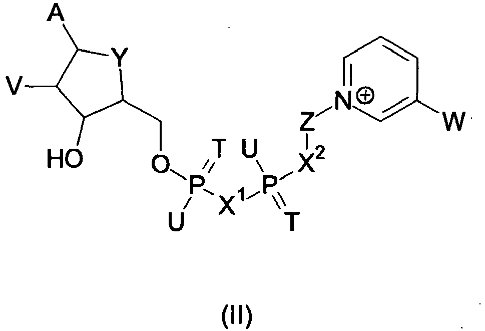 mit
- A =: Adenin oder ein Analog davon,
- T =: jeweils unabhängig O, S,
- U =: jeweils unabhängig OH, SH, BH₃⁻, BCNH₂⁻,
- V =: jeweils unabhängig OH oder eine Phosphatgruppe, oder zwei Gruppen, die eine cyclische Phosphatgruppe bilden;
- W =: COOR, CON(R)₂, COR, CSN(R)₂ mit R = jeweils unabhängig H oder C₁-C₂-Alkyl,
- X¹, X² =: jeweils unabhängig O, CH₂, CHCH₃, C(CH₃)₂, NH, NCH₃,
- Y =: NH, S, O, CH₂,
- Z =: ein linearer oder cyclischer organischer Rest ist, mit der Maßgabe, dass Z und der Pyridin-Rest nicht durch eine glycosidische Verbindung verknüpft sind, oder ein
Salz oder gegebenenfalls eine reduzierte Form davon.

In den Verbindungen der Formel (II) ist Z vorzugsweise ein linearer Rest mit 4-6 C-Atomen, vorzugsweise 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

Besonders bevorzugt ist Z ein gesättiger oder ungesättigter carbocyclischer oder heterocyclischer Fünfring, insbesondere eine Verbindung der allgemeinen Formel (III) 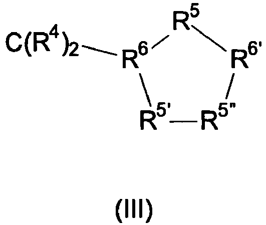
wobei zwischen R^{5'} und R^{5"} eine Einfach- oder Doppelbindung vorliegen kann, mit
R⁴ = jeweils unabhängig H, F, Cl, CH₃,
R⁵ =CR⁴₂,
wobei R^{5'} = O, S, NH, NC₁-C₂-Alkyl, CR⁴₂, CHOH, CHOCH₃, und
R^{5"} = CR⁴₂, CHOH, CHOCH₃, wenn zwischen R^{5'} und R^{5"} eine Einfachbindung vorliegt, und
wobei R^{5'}=R^{5"}=CR⁴, wenn zwischen R^{5'} und R^{5"} eine Doppelbindung vorliegt, und
R⁶, R^{6'} = jeweils unabhängig CH oder CCH₃.

In einer bevorzugten Ausführungsform enthalten die erfindungsgemäßen Verbindungen Adenin oder Adenin-Analoga, wie z.B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁-C₆-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxy-deoxyribose, 2'-Fluoro-deoxyribose, Hexitol, Altritol bzw. polycyclische Analoga, wie Bicyclo-, LNA- und Tricyclo-Zucker enthalten.

Insbesondere können in den Verbindungen der Formel (II) auch (Di-)-Phosphatsauerstoffe isotronisch ersetzt sein, wie z.B. O⁻ durch S⁻ bzw. BH₃⁻, O durch NH, NCH₃ bzw. CH₂ und =O durch =S.

In den erfindungsgemäßen Verbindungen der Formel (II) ist W vorzugsweise CONH₂ oder COCH₃.

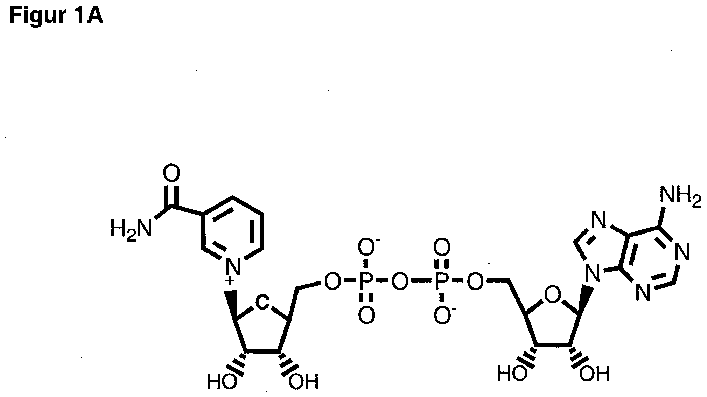

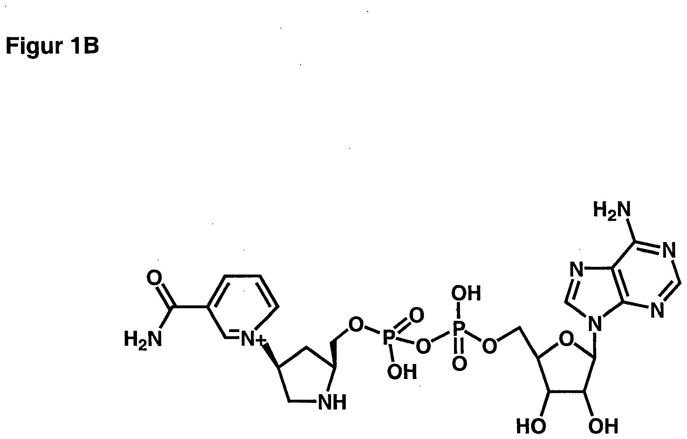

In den Gruppen der Formel (III) ist R⁵ vorzugsweise CH₂. Weiterhin ist bevorzugt, dass R^{5'} ausgewählt ist aus CH₂, CHOH und NH. In einer besonders bevorzugten Ausführungsform sind R^{5'} und R^{5"} jeweils CHOH. In noch einer weiteren bevorzugten Ausführungsform ist R^{5'} NH und R^{5"} CH₂. Spezifische Beispiele für bevorzugte stabilisierte Coenzyme sind in Figur 1A und B abgebildet.

In der am stärksten bevorzugten Ausführungsform handelt es sich bei dem stabilen Coenzym um carbaNAD.

Das erfindungsgemäße Verfahren ist zur Langzeitstabilisierung von Enzymen geeignet. Dies bedeutet, dass man das mit einem stabilen Coenzym stabilisierte Enzym, z.B. als Trockensubstanz, über eine Dauer von mindestens 2 Wochen, vorzugsweise von mindestens 4 Wochen und besonders bevorzugt von mindestens 8 Wochen lagert und wobei die Enzymaktivität vorzugsweise um weniger als 50 %, besonders bevorzugt weniger als 30 % und am meisten bevorzugt um weniger als 20 % bezüglich des Ausgangswerts der Enzymaktivität abnimmt.

Weiterhin umfasst das erfindungsgemäße Verfahren eine Lagerung des mit einem stabilen Coenzym stabilisierten Enzyms bei erhöhten Temperaturen, beispielsweise bei einer Temperatur von mindestens 20 °C, vorzugsweise von mindestens 25 °C und besonders bevorzugt von mindestens 30 °C. Die Enzymaktivität nimmt dabei vorzugsweise um weniger als 50 %, besonders bevorzugt weniger als 30 % und am meisten bevorzugt weniger als 20 % bezüglich ihres Ausgangswerts ab.

Durch die erfindungsgemäße Stabilisierung ist es möglich, das mit einem stabilen Coenzym stabilisierte Enzym auch ohne Trocknungsreagenz für eine lange Zeit, wie oben angegeben, und gegebenenfalls bei hohen Temperaturen, wie oben angegeben, zu lagern. Weiterhin kann das stabilisierte Enzym auch bei einer hohen relativen Luftfeuchtigkeit, z.B. einer relativen Luftfeuchtigkeit von mindestens 50 % gelagert werden, wobei die Enzymaktivität vorzugsweise um weniger als 50 %, besonders bevorzugt weniger als 30 % und am meisten bevorzugt weniger als 20 % bezüglich des Ausgangswerts abnimmt.

Die Lagerung des mit einem stabilen Coenzym stabilisierten Enzyms kann einerseits als Trockensubstanz und andererseits in Flüssigphase erfolgen. Bevorzugt erfolgt die Lagerung des stabilisierten Enzyms auf oder in einem Testelement, das zur Bestimmung eines Analyten geeignet ist. Das mit einem stabilen Coenzym stabilisierte Enzym ist dabei vorzugsweise Bestandteil eines Nachweisreagenz, welches gegebenenfalls noch weitere Bestandteile wie etwa Salze, Puffer, etc. enthalten kann. Vorzugsweise ist das Nachweisreagenz dabei frei von einem Mediator.

Das mit einem stabilen Coenzym stabilisierte Enzym kann zum Nachweis von Analyten, beispielsweise Parametern in Körperflüssigkeiten wie etwa Blut, Serum, Plasma oder Urin bzw. in Abwasserproben oder Lebensmitteln eingesetzt werden.

Als Analyten können beliebige biologische oder chemische Substanzen bestimmt werden, die durch eine Redoxreaktion nachgewiesen werden können, z.B. Substanzen, bei denen es sich um Substrate eines Coenzymabhängigen Enzyms handelt oder Coenzym-abhängige Enzyme selbst. Bevorzugte Beispiele für Analyten sind Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH), Kohlendioxid etc. Bevorzugt ist der Analyt Glucose.

Die Veränderung des stabilen Coenzyms durch Reaktion mit dem Analyten kann grundsätzlich auf beliebige Art und Weise nachgewiesen werden. Hier können grundsätzlich alle aus dem Stand der Technik bekannten Methoden zum Nachweis enzymatischer Reaktionen eingesetzt werden. Vorzugsweise wird jedoch die Veränderung des Coenzyms durch optische Methoden nachgewiesen. Optische Nachweismethoden umfassen beispielsweise die Messung von Absorption, Fluoreszenz, Circulardichroismus (CD), optische Rotationsdispersion (ORD), Refraktometrie etc.

Ein optisches Nachweisverfahren, welches im Rahmen der vorliegenden Anmeldung bevorzugt Anwendung findet, ist die Photometrie. Zur photometrischen Messung einer Veränderung des Coenzyms infolge Umsetzung mit dem Analyten bedarf es indessen der zusätzlichen Anwesenheit mindestens eines Mediators, welcher die Reaktivität des reduzierten Coenzyms erhöht und eine Übertragung von Elektronen auf einen geeigneten optischen Indikator oder ein optisches Indikatorsystem ermöglicht.

Als Mediatoren, welche für die Zwecke der vorliegenden Erfindung geeignet sind, kommen u.a. Nitrosoaniline, wie beispielsweise [(4-Nitrosophenyl)imino]dimethanol-Hydrochlorid, Chinone, wie beispielsweise Phenanthrenchinone, Phenanthrolinchinone oder Benzo[h]-chinolinchinone, Phenazine, wie beispielsweise 1-(3-Carboxypropoxy)-5-ethylphenazinium-trifluormethansulfonat, oder/und Diaphorase (EC 1.6.99.2) in Frage. Bevorzugte Beispiele für Phenanthrolinchinone umfassen 1,10-Phenanthrolin-5,6-chinone, 1,7-Phenanthrolin-5,6-chinone, 4,7-Phenanthrolin-5,6-chinone sowie deren N-alkylierte oder N,N'-dialkylierte Salze, wobei im Falle N-alkylierter bzw. N,N'-dialkylierter Salze Halogenide, Trifluormethansulfonat oder andere die Löslichkeit erhöhende Anionen als Gegenion bevorzugt sind.

Als optischer Indikator oder als optisches Indikatorsystem kann eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden photometrischen Verfahrens erfolgen. Vorzugsweise werden Heteropolysäuren, und insbesondere 2,18-Phosphormolybdänsäure, als optische Indikatoren verwendet, die zum entsprechenden Heteropolyblau reduziert werden.

Besonders bevorzugt wird die Veränderung des Coenzyms durch Messung der Fluoreszenz nachgewiesen. Die Fluoreszenzmessurig ist hochsensitiv und ermöglicht den Nachweis selbst geringer Konzentrationen des Analyten in miniaturisierten Systemen.

Alternativ kann die Veränderung des Coenzyms auch elektrochemisch unter Verwendung eines geeigneten Testelements, wie beipielsweise eines elektrochemischen Teststreifens, nachgewiesen werden. Voraussetzung hierfür ist wiederum die Verwendung geeigneter Mediatoren, welche vom reduzierten Coenzym durch Übertragung von Elektronen in eine reduzierte Form überführt werden können. Die Bestimmung des Analyten erfolgt über eine Messung des zur Reoxidation des reduzierten Mediators benötigten Stromes, welcher mit der Konzentration des Analyten in der Probe korreliert. Beispiele für Mediatoren, welche für elektrochemische Messungen verwendet werden können, umfassen insbesondere die vorstehend erwähnten, für photometrische Messungen eingesetzten Mediatoren.

Zum Nachweis eines Analyten kann ein Flüssigtest verwendet werden, wobei das Reagenz z.B. in Form einer Lösung oder Suspension in einer wässrigen oder nichtwässrigen Flüssigkeit oder als Pulver oder Lyophilisat vorliegt. Es kann jedoch auch ein Trockentest verwendet werden, wobei das Reagenz auf einem Träger, einem Teststreifen, aufgebracht ist. Der Träger kann beispielsweise einen Teststreifen, umfassend ein saugfähiges oder/und quellbares Material, umfassen, das von der zu untersuchenden Probeflüssigkeit benetzt wird.

Ein besonders bevorzugtes Testformat, umfasst die Verwendung des Enzyms Glucose-Dehydrogenase mit einem stabilen NAD-Derivat zum Nachweis von Glucose, wobei ein Derivat des reduzierten Coenzyms NADH gebildet wird. Der Nachweis von NADH erfolgt durch optische Methoden, z.B. durch photometrische oder fluorometrische Bestimmung nach UV-Anregung. Ein besonders bevorzugtes Testsystem ist in US 2005/0214891 beschrieben, auf das hier ausdrücklich Bezug genommen wird.

Noch ein weiterer Gegenstand der vorliegenden Erfindung ist ein mit einem stabilen Coenzym stabilisiertes Enzym gemäß Anspruch 19, wobei das stabilisierte Enzym bei einer Lagerung von vorzugsweise mindestens 2 Wochen, besonders bevorzugt mindestens 4 Wochen und am meisten bevorzugt mindestens 8 Wochen bei einer Temperatur von vorzugsweise mindestens 20 °C, besonders bevorzugt mindestens 25 °C und am meisten bevorzugt mindestens 30 °C, gegebenenfalls bei hoher Luftfeuchtigkeit und ohne Trockenreagenz eine Abnahme der enzymatischen Aktivität von weniger als 50 %, vorzugsweise weniger als 30 % und am meisten bevorzugt weniger als 20 % gegenüber dem Ausgangswert aufweist.

Noch ein weiterer Gegenstand der Erfindung ist ein Nachweisreagenz gemäß Anspruch 20 zur Bestimmung eines Analyten, welches ein mit einem stabilen Coenzym stabilisiertes Enzym, wie zuvor angegeben, enthält. Außerdem betrifft die Erfindung ein Testelement gemäß Anspruch 21, welches ein erfindungsgemäßes stabilisiertes Enzym bzw. ein erfindungsgemäßes Nachweisreagenz enthält. Das Nachweisreagenz bzw. das Testelement können durch Durchführung von Trocken- oder Flüssigtests geeignet sein. Bevorzugt ist das Testelement ein Teststreifen zum fluorometrischen oder photometrischen Nachweis eines Analyten. Ein solcher Teststreifen enthält das mit einem stabilen Coenzym stabilisierte Enzym immobilisiert auf einem saugfähigen oder/und quellbaren Material, wie etwa Cellulose, Kunstoffe, etc.

Die Erfindung soll durch die nachfolgenden Figuren und Beispiele näher erläutert werden:

### Beschreibung der Figuren

**Figur 1A****:** Darstellung des stabilen Coenzym carba-NAD (cNAD).
**Figur 1B****:** Darstellung des stabilen Coenzyms Pyrrolidinyl-NAD.
**Figur 2****:** Darstellung der Ergebnisse der Enzymkinetik von Glucose-Dehydrogenase in Gegenwart von NAD bzw. von Glucose-Dehydrogenase in Gegenwart von cNAD vor und nach Lagerung.
   **2A:** Kinetik von GlucDH in Gegenwart von NAD nach 1 Tag.
   **2B:** Kinetik von GlucDH in Gegenwart von cNAD nach 1 Tag.
   **2C:** Kinetik von GlucDH in Gegenwart von NAD nach 5 Wochen Lagerung bei 32 °C und 85 % relativer Luftfeuchtigkeit.
   **2D:** Kinetik von GlucDH in Gegenwart von cNAD nach 5 Wochen Lagerung bei 32 °C und 85 % relativer Luftfeuchtigkeit.

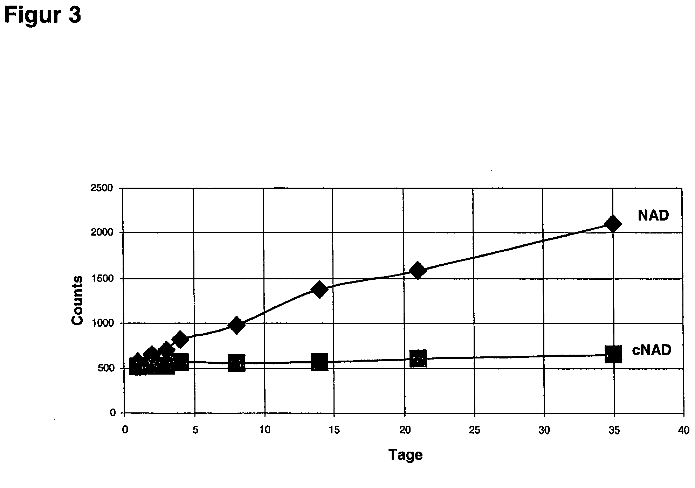

**Figur 3****:** Vergleich der Blankwerte von Glucose-Dehydrogenase in Gegenwart von NAD bzw. von GlucDH in Gegenwart von cNAD über einen Zeitraum von bis zu 5 Wochen bei 32 °C und 85 % Luftfeuchtigkeit.

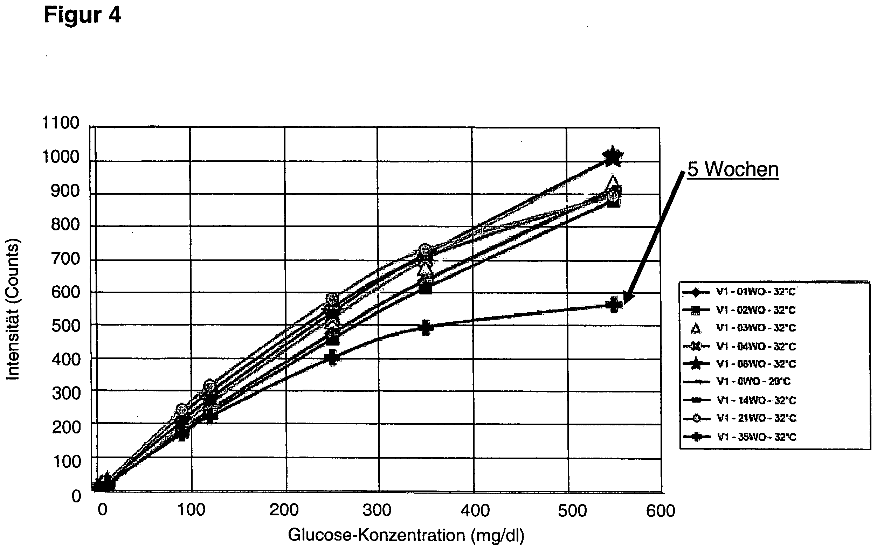

**Figur 4****:** Darstellung verschiedener Funktionskurven von Glucose-Dehydrogenase nach Lagerung von Glucose-Dehydrogenase in Gegenwart von NAD bei 32 °C und 85 % Luftfeuchtigkeit. Die Lagerungsdauer variierte zwischen 1 Tag und 5 Wochen.

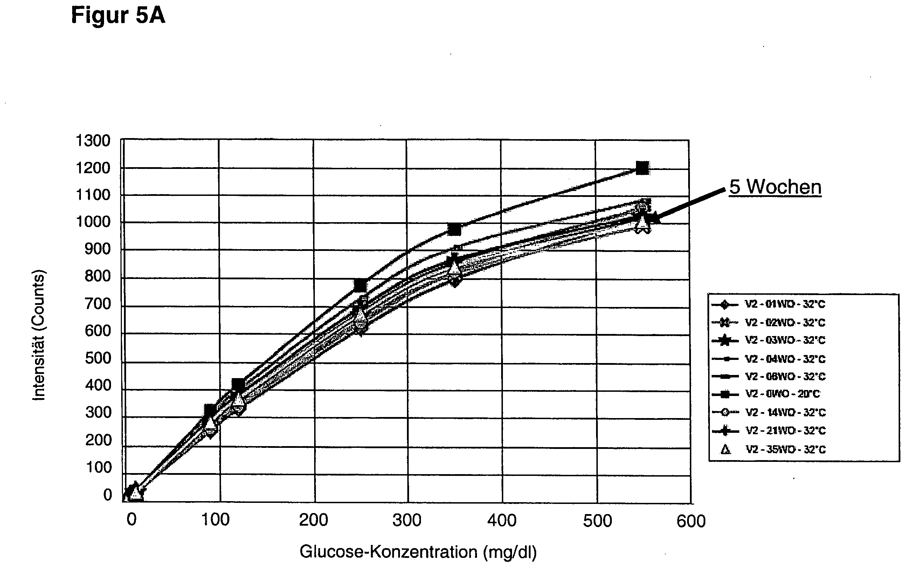

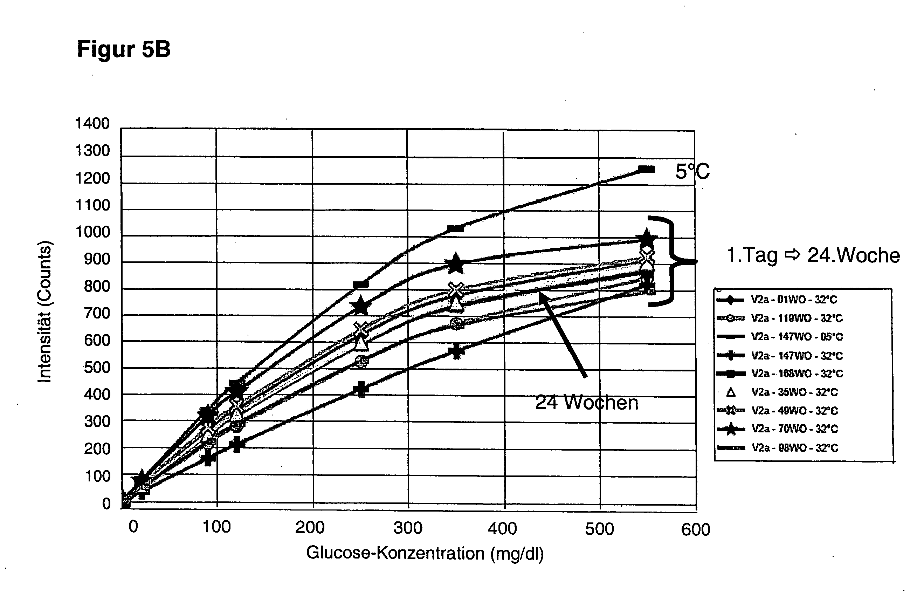

**Figur 5****:** Darstellung verschiedener Funktionskurven von Glucose-Dehydrogenase nach Lagerung von Glucose-Dehydrogenase in Gegenwart von cNAD bei 32 °C und 85 % Luftfeuchtigkeit. Die Lagerungsdauer variierte zwischen 1 Tag und 5 Wochen (Figur 5A) bzw. zwischen 1 Tag und 24 Wochen (Figur 5B).

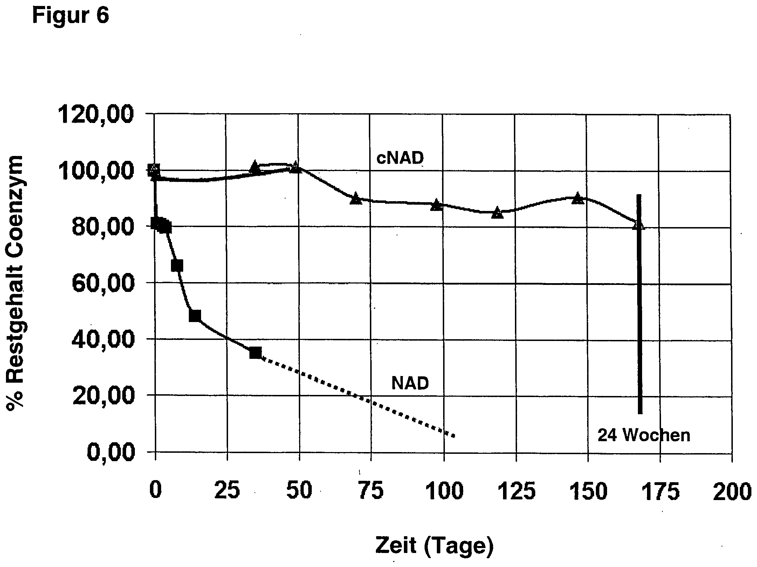

**Figur 6****:** Darstellung des Restgehaltes an NAD bzw. cNAD nach Lagerung von Glucose-Dehydrogenase in Gegenwart von NAD bzw. cNAD über 24 Wochen bei 32 °C und 85 % Luftfeuchtigkeit.

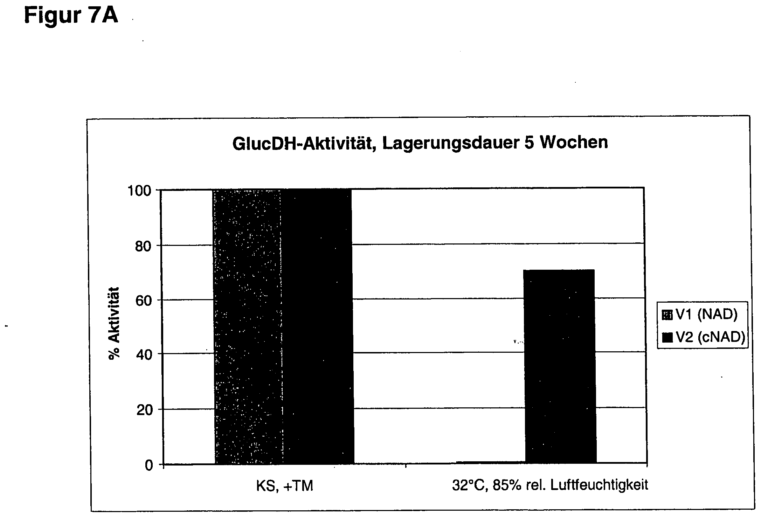

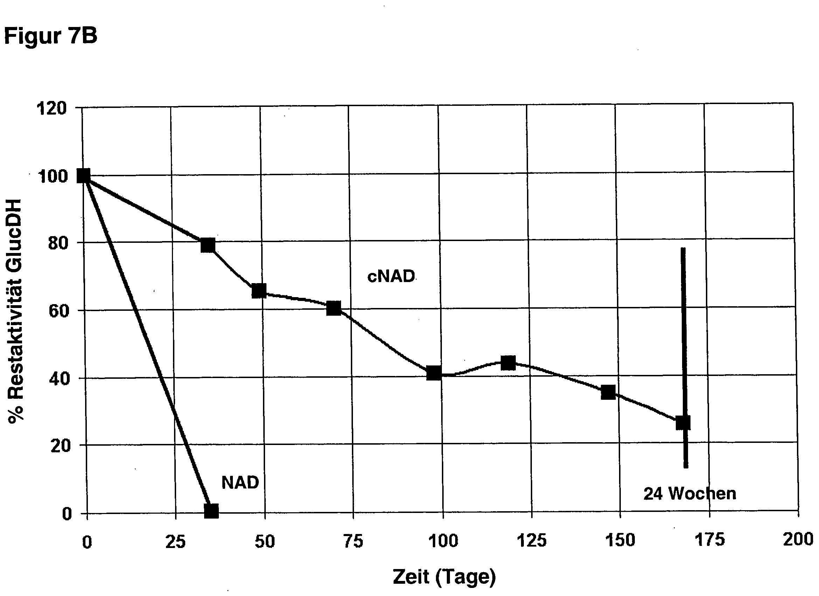

**Figur 7**: Darstellung der GlucDH-Aktivität nach Lagerung von Glucose-Dehydrogenase in Gegenwart von NAD bzw. cNAD über 5 Wochen (Figur 7A) bzw. 24 Wochen (Figur 7B) bei 32 °C und 85 % Luftfeuchtigkeit.

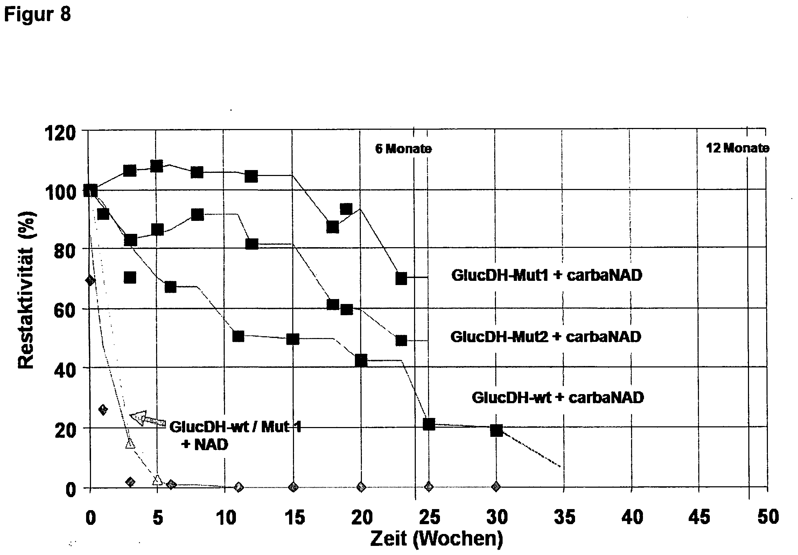

**Figur 8****:** Darstellung der GlucDH-Aktivität nach Lagerung von Glucose-Dehydrogenase (GlucDH-wt), der Doppelmutante GlucDH_E96G_E170K (GlucDH-Mut1) und der Doppelmutante GlucDH_E170K_K252L (GlucDH-Mut2) über einen Zeitraum von 25 Wochen in Gegenwart von NAD bzw. cNAD bei 32°C und 83% relativer Luftfeuchtigkeit.

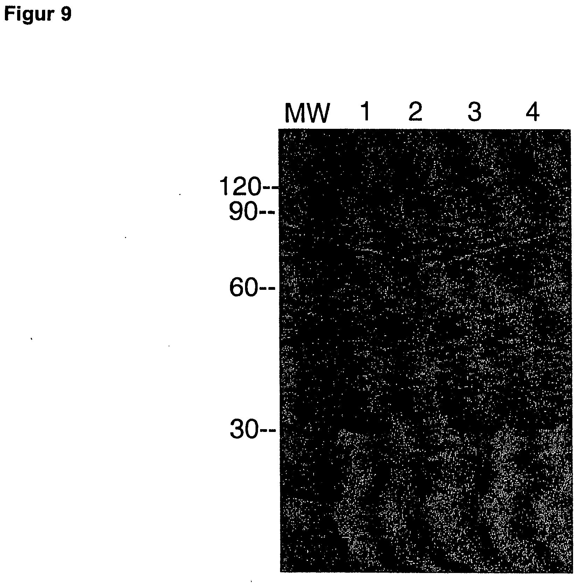

**Figur 9****:** Gelelektrophoretische Analyse von Glucose-Dehydrogenase nach Lagerung in Gegenwart von NAD bzw. cNAD. Testbedingungen: MW, 10-220 kDa-Marker; 1: GlucDH/NAD, 5 Wochen bei 6 °C; 2: GlucDH/NAD, 5 Wochen bei 32 °C/85 % relative Luftfeuchtigkeit; 3: GlucDH/cNAD, 5 Wochen bei 6 °C; 4: GlucDH/cNAD, 5 Wochen bei 32 °C/85 % relative Luftfeuchtigkeit.

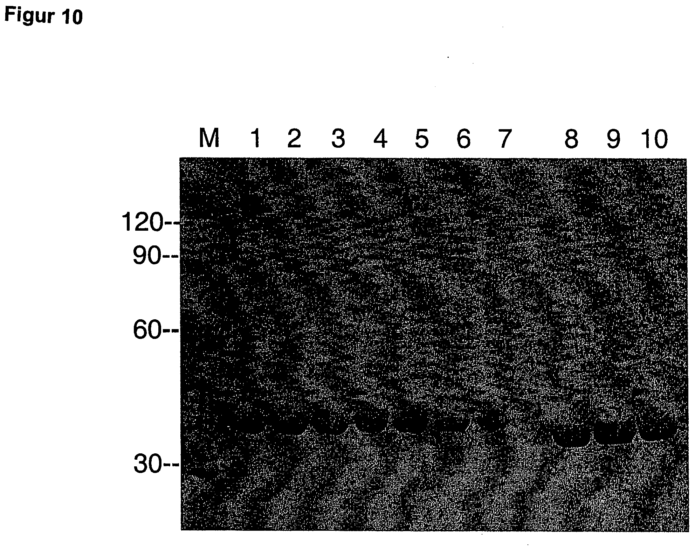

**Figur 10****:** Gelelektrophoretische Analyse von Glucose-Dehydrogenase nach Lagerung bei 50 °C in Gegenwart von NAD bzw. cNAD. Testbedingungen: MW, 10-220 kDa-Marker; 1: GlucDH 8,5 mg/ml, NAD, 0 Stunden; 2: GlucDH 8,5 mg/ml, NAD, 22 Stunden; 3: GlucDH 8,5 mg/ml, NAD, 96 Stunden; 4: GlucDH 8,5 mg/ml, NAD, 118 Stunden; 5: GlucDH 8,5 mg/ml, NAD, 140 Stunden; 6: GlucDH 8,5 mg/ml, NAD, 188 Stunden; 7: GlucDH 8,5 mg/ml, NAD, 476 Stunden; 8: GlucDH 8,5 mg/ml, cNAD, 0 Stunden; 9: GlucDH 8,5 mg/ml, cNAD, 188 Stunden; 10: GlucDH 8,5 mg/ml, cNAD, 476 Stunden.

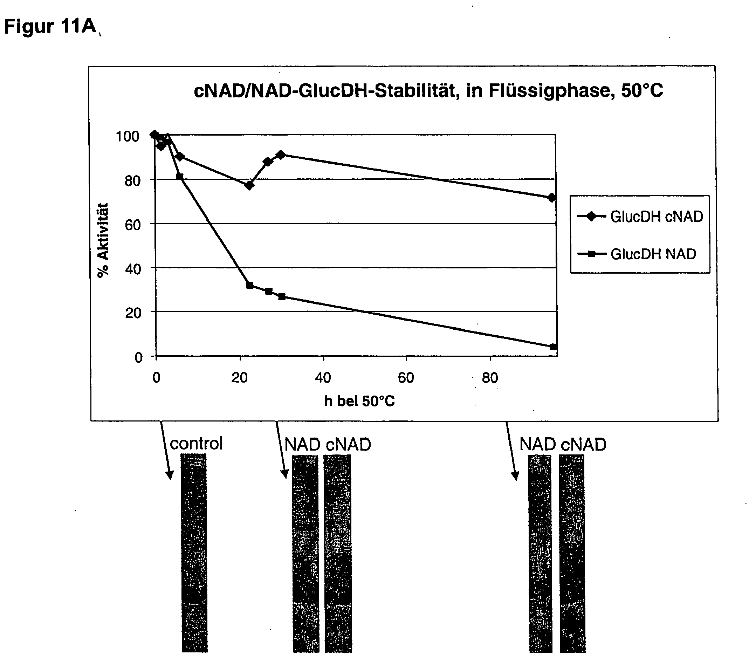

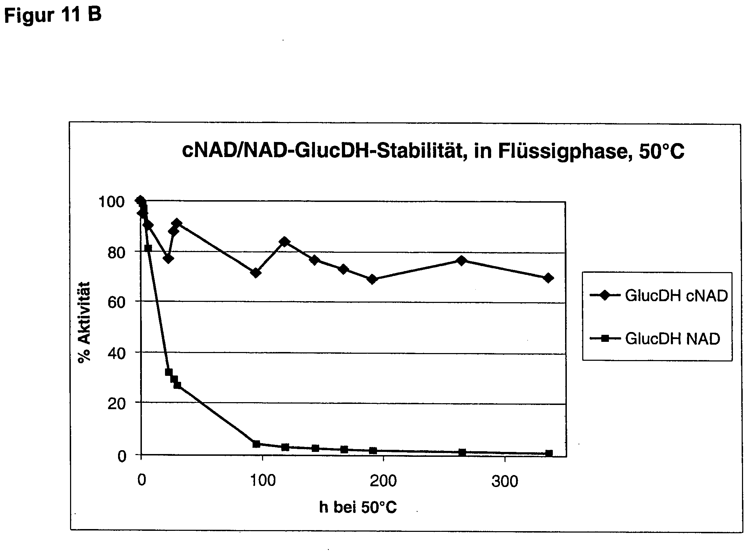

**Figur 11****:** Darstellung der Stabilität von Glucose-Dehydrogenase in Gegenwart von NAD bzw. cNAD in Flüssigphase bei 50 °C über einen Zeitraum von 4 Tagen (Figur 11A) bzw. 14 Tagen (Figur 11B). Testbedingungen: GlucDH 10 mg/ml; NAD bzw. cNAD 12 mg/ml; Puffer: 0,1 M Tris, 1,2 M NaCl, pH 8,5; Temperatur 50 °C.

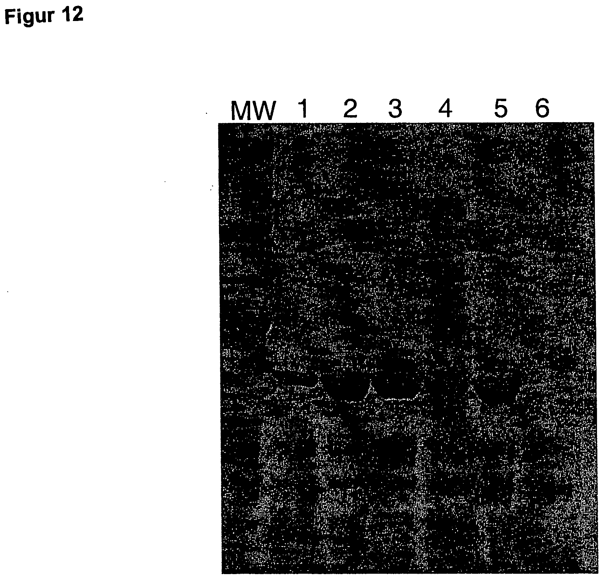

**Figur 12****:** Gelelektrophoretische Analyse von Alkohol-Dehydrogenase aus Hefe nach Lagerung in Gegenwart von NAD bzw. cNAD. Testbedingungen: MW, 10-220 kDa-Marker; 1: ADH, 65 Stunden bei 6 °C; 2: ADH/cNAD, 65 Stunden bei 6 °C; 3: ADH/NAD, 65 Stunden bei 6 °C; 4: ADH, 65 Stunden bei 35 °C; 5: ADH/cNAD, 65 Stunden bei 35 °C; 6: ADH/NAD, 65 Stunden bei 35 °C.

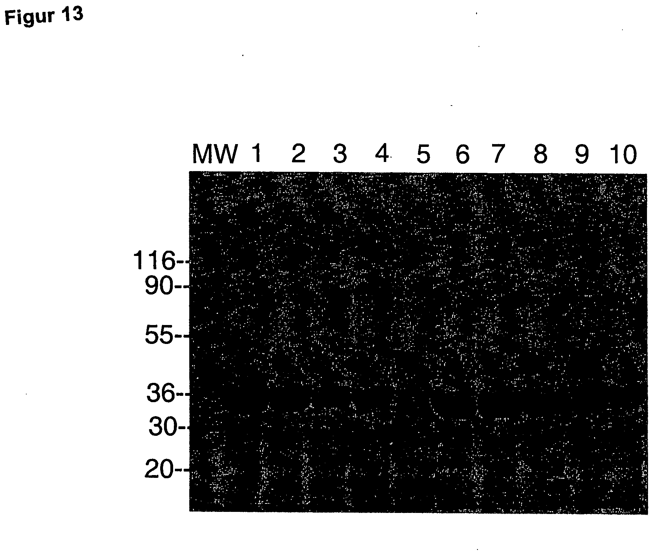

**Figur 13****:** Gelelektrophoretische Analyse von Alkohol-Dehyrogenase aus Hefe nach Lagerung bei 35 °C in Gegenwart von NAD bzw. cNAD. Testbedingungen: MW, 10-220 kDa-Marker; 1: ADH/NAD, 0 Tage; 2: ADH/NAD, 1 Tag; 3: ADH/NAD, 2 Tage; 4: ADH/NAD, 3 Tage; 5: ADH/NAD, 5 Tage; 6: ADH/cNAD, 0 Tage; 7: ADH/cNAD, 1 Tag; 8: ADH/cNAD, 2 Tage; 9: ADH/cNAD, 3 Tage; 10: ADH/cNAD, 6 Tage.

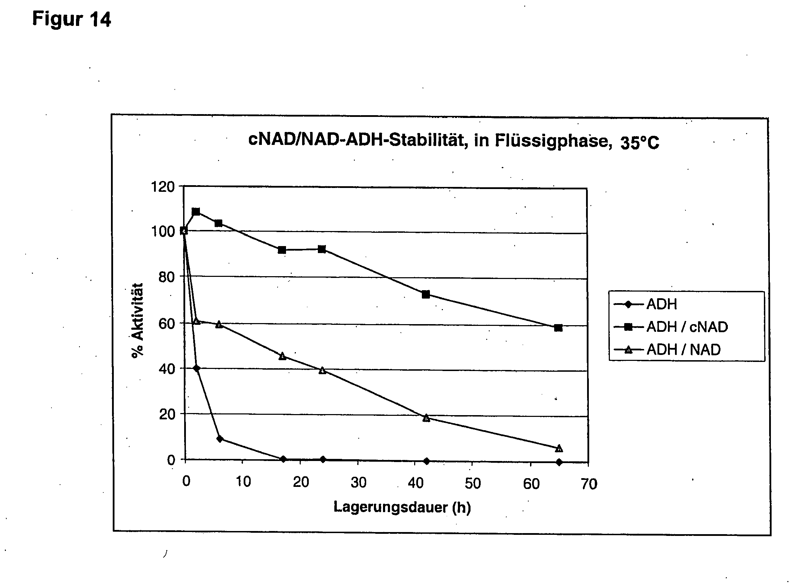

**Figur 14****:** Darstellung der Stabilität von Alkohol-Dehydrogenase aus Hefe in Gegenwart von NAD bzw. cNAD in Flüssigphase bei 35 °C über einen Zeitraum von 65 Stunden. Testbedingungen: ADH 5 mg/ml; NAD bzw. cNAD 50 mg/ml; Puffer: 75 mM Na₄P₂O₇, Glycin, pH 9,0; Temperatur 35 °C.

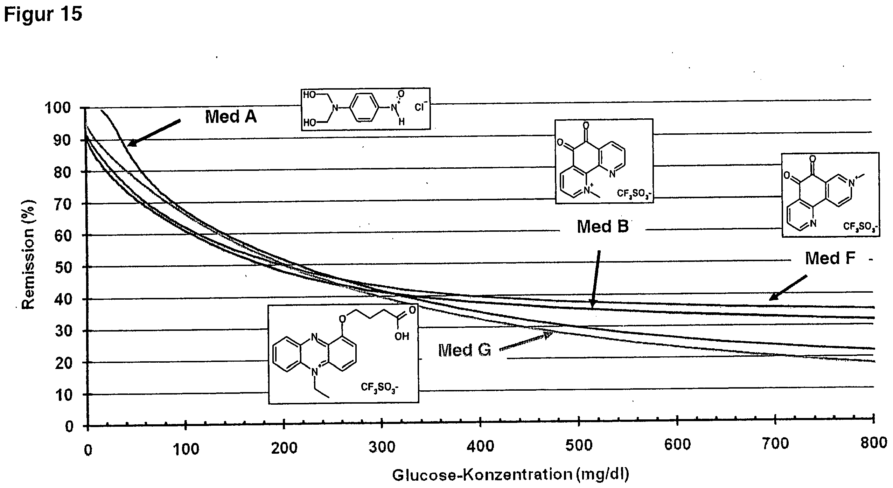

**Figur 15****:** Darstellung verschiedener Funktionskurven von Glucose-Dehydrogenase nach 11 Wochen Lagerung in Gegenwart von NAD und unterschiedlicher Mediatoren bei Raumtemperatur.

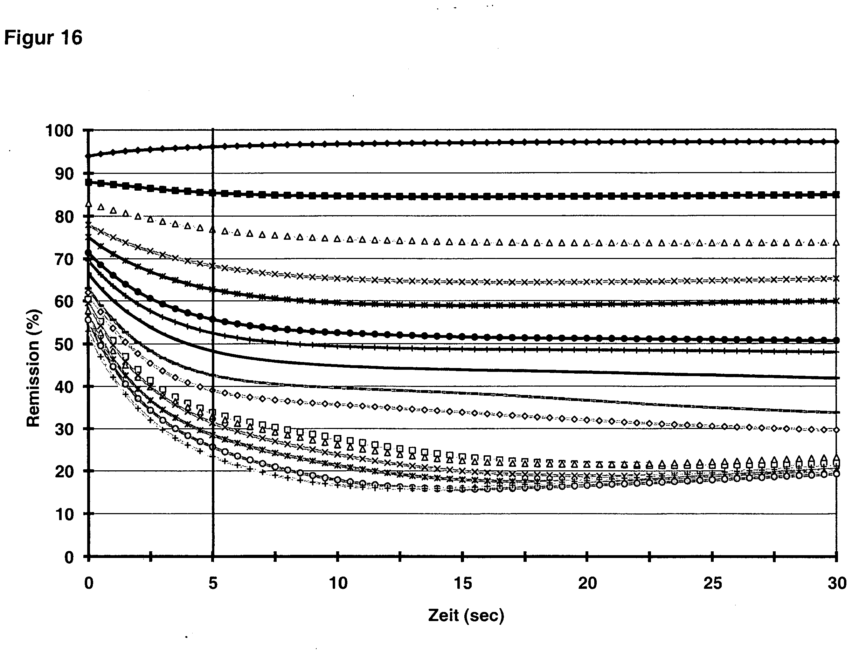

**Figur 16****:** Darstellung der Ergebnisse der Enzymkinetik von Glucose-Dehydrogenase in Gegenwart von NAD und 1-(3-Carboxypropoxy)-5-ethylphenazinium-trifluormethansulfonat bei verschiedenen Glucose-Konzentrationen.

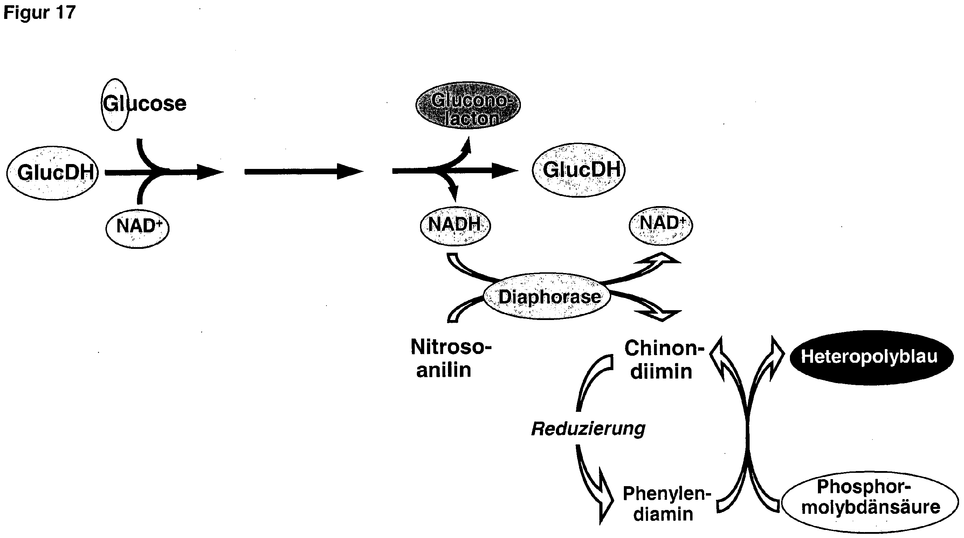

**Figur 17****:** Schematische Darstellung des Glucose-Nachweises mit GlucDH als Enzym und Diaphorase als Mediator.

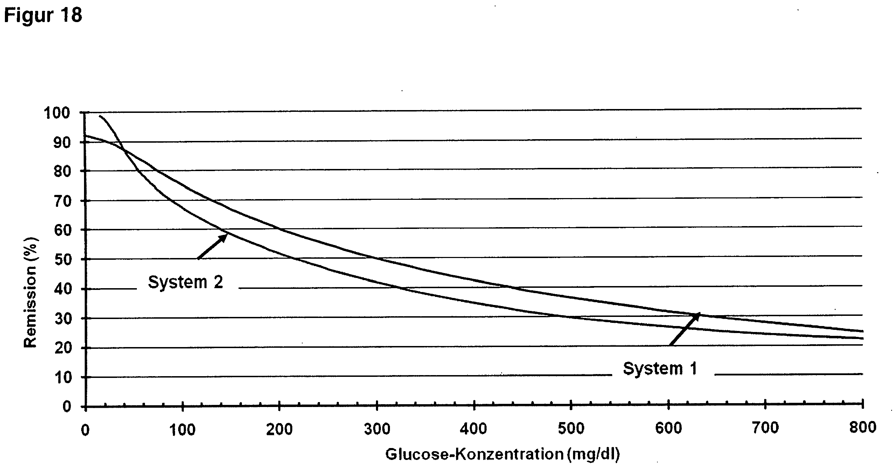

**Figur 18****:** Darstellung der Funktionskurven von Glucose-Dye-Oxidoreduktase (GlucDOR) in Gegenwart von Pyrrolochinolinchinon (PQQ) und [(4-Nitrosophenyl)imino]dimethanol-Hydrochlorid als Mediator bzw. von Glucose-Dehydrogenase in Gegenwart von NAD und Diaphorase/[(4-Nitrosophenyl)imino]dimethanol-Hydrochlorid als Mediator.

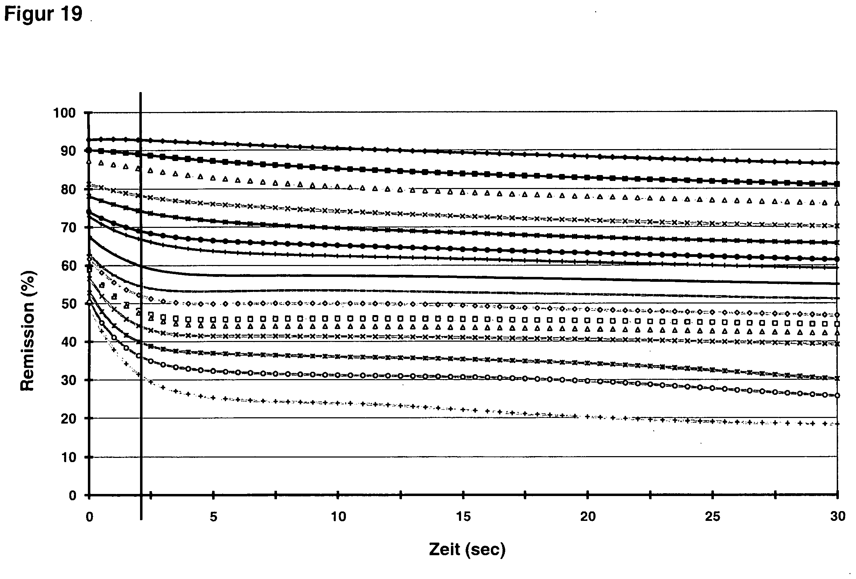

**Figur 19****:** Darstellung der Ergebnisse der Enzymkinetik von Glucose-Dehydrogenase in Gegenwart von NAD und Diaphorase bei verschiedenen Glucose-Konzentrationen.

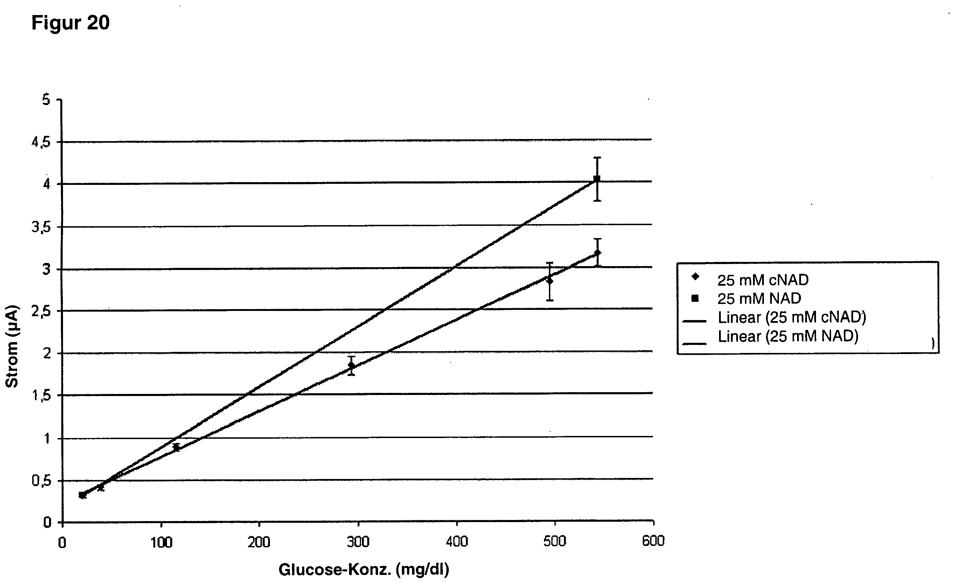

**Figur 20****:** Darstellung des in Abhängigkeit von der Glucose-Konzentration gemessenen Stromes bei elektrochemischer Bestimmung von Glucose unter Verwendung von Glucose-Dehydrogenase in Gegenwart von NAD bzw. cNAD. Testbedingungen: 25 mM NAD bzw. cNAD; 2.5 Sekunden Verzögerung; 5 Sekunden Messzeit.

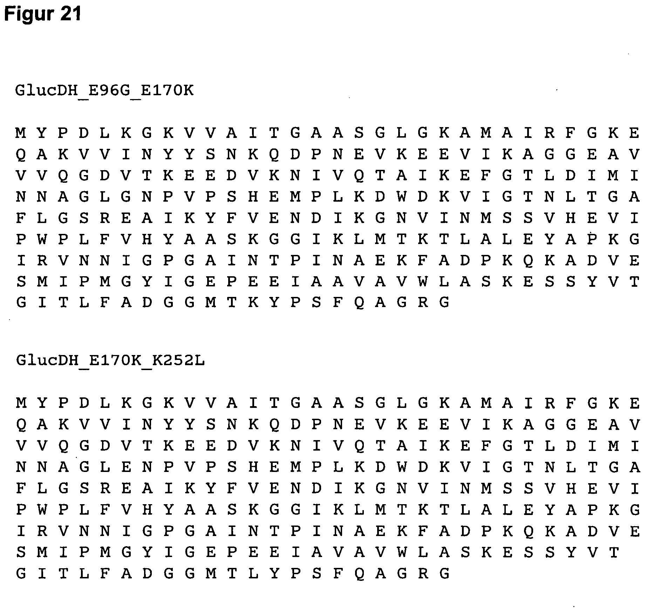

**Figur 21****:** Darstellung der Aminosäuresequenzen der Glucose-Dehydrogenase-Doppelmutanten GlucDH_E96G_E170K und GlucDH_E170K_K252L.

### Beispiel 1

Der Glucose-spezifischen GlucDH wurden carba-NAD (Fig. 1A) oder NAD zugesetzt. Diese Rezepturen wurden jeweils auf Pokalonfolie (Lonza) aufgetragen und nach Trocknung unter warmen und feuchten Bedingungen (32 °C, 85 % relative Luftfeuchtigkeit) eingelagert. Anschließend wurden in regelmäßigen Abständen die Reaktionskinetik sowie die Funktionskurve bestimmt. Parallel wurde zu den jeweiligen Messterminen eine cNAD/NAD-Analytik sowie eine Bestimmung der Restaktivität des Enzyms vorgenommen.

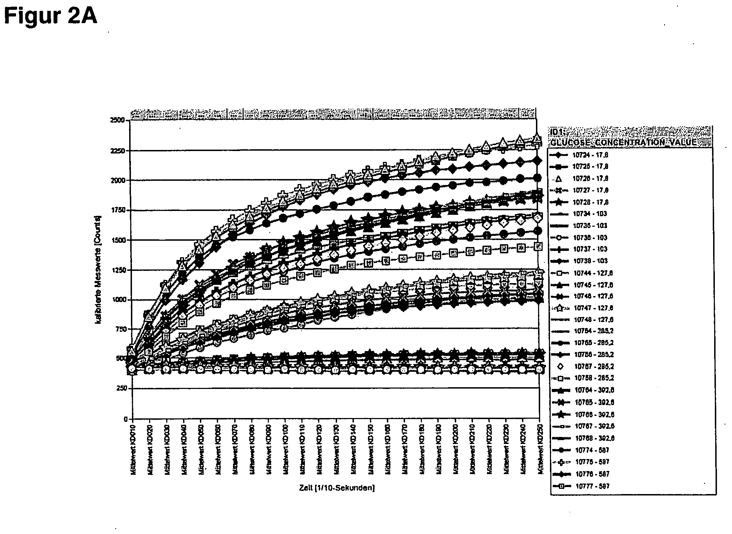

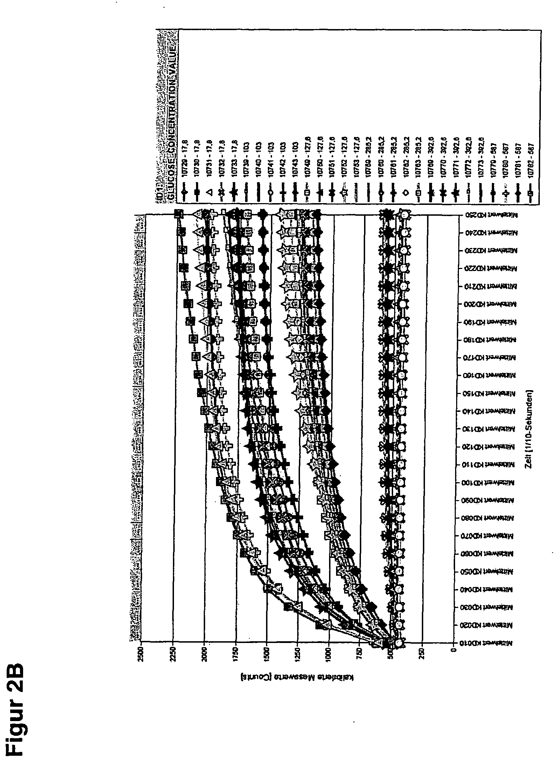

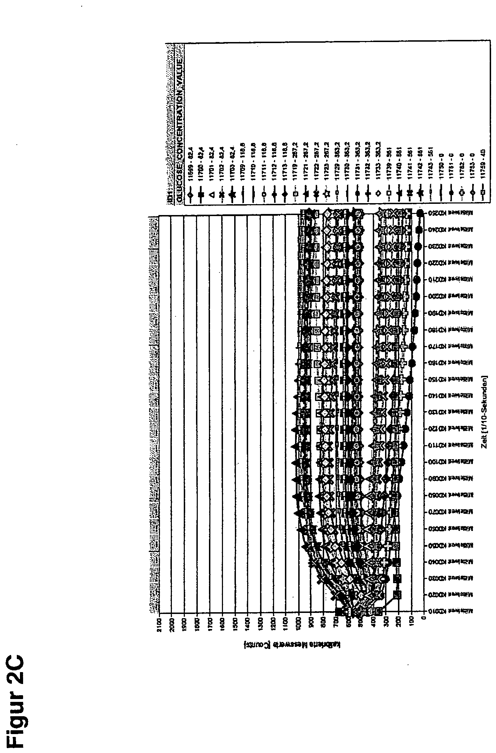

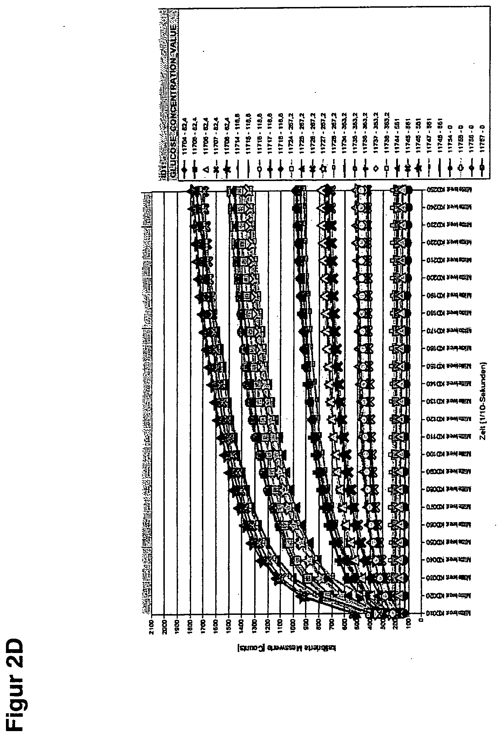

Die am ersten Tag bestimmten Kinetik-Kurven für NAD (Figur 2A) und cNAD (Figur 2B) sind vergleichbar und zeigen auch einen ähnlichen Hub in der Glucoseabhängigkeit. Nach 5 Wochen ist jedoch ein deutlicher Unterschied in den Kinetik-Kurven zu erkennen. Während die Kinetik für NAD (Figur 2C) stark in ihrer Dynamik nachlässt, bleibt die Kinetik des mit cNAD stabilisierten Enzyms praktisch unverändert (Figur 2D).

Auch in den Blankwerten (Trockenleerwert vor Auftrag einer Blutprobe) zeigt sich ein deutlicher Unterschied, wie aus Figur 3 ersichtlich ist. Der Anstieg des Trockenleerwerts bei NAD ist auf die Bildung von fluoreszierenden Teilchen zurückzuführen (Oppenheimer (1982), Supra). Überraschenderweise geschieht dies nicht bei cNAD.

Die unterschiedliche Stabilität von Glucose-Dehydrogenase in Gegenwart von NAD bzw. cNAD ist auch aus einem Vergleich der Figuren 4 und 5 ersichtlich. Nach 5 Wochen liegt die Funktionskurve bei dem mit cNAD stabilisierten Enzym noch in der Schar der voherigen Messungen (Figur 5A), während bei dem mit NAD versetzten Enzym (Figur 4) die Kurve bei höheren Konzentrationen abknickt, was ein typisches Zeichen für zu geringe Mengen an Enzym/Coenzym ist. Figur 5B zeigt verschiedene Funktionskurven der mit cNAD stabilisierten Glucose-Dehydrogenase über einen Zeitraum von 24 Wochen. In diesem Zusammenhang wird deutlich, dass sich die Funktion des Enzyms bei hohen Glucose-Konzentrationen über den gesamten Zeitraum hinweg nur geringfügig verändert und nach 24 Wochen etwa dem nach 5 Wochen erhaltenen Wert entspricht.

Die Beziehung zwischen Struktur des Coenzyms und dessen Stabilität über einen vorbestimmten Zeitraum ergibt sich aus Figur 6. Demnach beträgt der Restgehalt an cNAD in einem Glucose-Nachweisreagenz nach 24 Wochen Lagerung (bei 32 °C und 85 % relativer Luftfeuchtigkeit) noch etwa 80 % des Ausgangswertes, während sich der Gehalt an NAD in einem mit NAD stabilisierten Glucose-Nachweisreagenz bereits nach 5 Wochen auf etwa 35 % des Ausgangswertes verringert und gemäß Extrapolation nach etwa 17 Wochen auf null reduziert.

Vollkommen überraschend ist das Ergebnis der Restaktivitätsbestimmung des aktiven Enzyms GlucDH nach 5 Wochen bei 32 °C und 85 % relative Luftfeuchtigkeit (Figur 7A). Das mit NAD stabilisierte Enzym weist nur noch eine extrem geringe Enzymaktivität auf (0,5 %), während das mit cNAD stabilisierte Enzym noch eine Restaktivität von 70 % besitzt (jeweils im Vergleich zu den mit Trockenmittel (TM) im Kühlschrank (KS) eingelagerten Proben). Nach 24 Wochen bei 32 °C und 85 % relativer Luftfeuchtigkeit (Figur 7B) beträgt die Restaktivität des Enzyms bei Stabilisierung mit cNAD noch immer etwa 25 %.

Wird anstelle des Wildtyp-Enzyms (aus Bacillus subtilis) eine Mutante verwendet, so kann die Restaktivität von GlucDH noch weiter gesteigert werden. Nach 24 Wochen Lagerung bei 32 °C und 85 % relativer Luftfeuchtigkeit in Gegenwart von cNAD beträgt die Restaktivität einer Mutante GlucDH_E96G_E170K mit Aminosäuresubstitutionen Glutaminsäure → Glycin an Position 96 und Glutaminsäure → Lysin an Position 170 (GlucDH-Mut1) des Wildtyp-Enzyms etwa 70 %, während die Restaktivität einer Mutante GlucDH_E170K_K252L mit Aminosäuresubstitutionen Glutaminsäure → Lysin an Position 170 und Lysin → Leucin an Position 252 (GlucDH-Mut2) bei etwa 50 % liegt (Figur 8).

Auch die gelelektrophoretische Analyse von Glucose-Dehydrogenase im SDS-Gel (Figuren 9 und 10) zeigt deutlich den Unterschied zwischen einer Lagerung in Anwesenheit von NAD bzw. cNAD. Während das Enzym nach 5 Wochen Lagerung bei 32 °C und 85 % relativer Luftfeuchtigkeit in Gegenwart von cNAD noch als Bande mit der erwarteten Mobilität zu erkennen ist, ist das in Gegenwart von NAD gelagerte Enzym vollkommen verschwunden (Figur 9). Gleichzeitig ist aus Figur 10 ersichtlich, dass die Bande des mittels NAD stabilisierten und bei 50 °C gelagerten Enzyms mit zunehmender Lagerungsdauer schwächer wird und nach 476 Stunden nahezu verschwunden ist, während die entsprechende Bande des in Gegenwart von cNAD gelagerten Enzyms gegenüber einer zu Beginn des Experiments detektierten Bande eine lediglich geringfügige Änderung zeigt.

Dieses Ergebnis läßt sich auch bei Lagerung in flüssiger Phase bestätigen. (Figuren 11A und 11B). Nach 95 Stunden bei 50 °C liegt die Restaktivität von Glucose-Dehydrogenase in Gegenwart des nativen Coenzyms NAD bei ≈ 5 %, während die Restaktivität der GlucDH in Gegenwart des artifiziellen Coenzyms cNAD bei 75 % liegt (Figur 11A). Nach 336 Stunden Lagerung bei 50 °C beträgt die Restaktivität des mit NAD stabilisierten Enzyms nur mehr etwa 1 %; für das in Gegenwart von cNAD gelagerte Enzym wird eine Restaktivität von immer noch etwa 70 % beobachtet. Die entsprechenden SDS-Gele zeigen ebenfalls eine Veränderung der GlucDH-Bande in Gegenwart des nativen Coenzyms NAD: es zeigen sich neue Banden bei höheren Molmassen und eine Verschiebung der 30 kDa-Bande.

Insgesamt ist es ein höchst überraschendes Ergebnis, dass die Stabilisierung des Cofaktors gleichzeitig eine Stabilisierung des Enzyms bewirkt - und dies nicht allein durch den kooperativen Effekt des besseren Zusammenhaltes des Enzyms. Der Zerfall des Cofaktors NAD hat einen negativen Effekt auf die Stabilität des Enzyms GlucDH und beschleunigt sogar dessen Inaktivierung. Der Austausch von nativem NAD durch künstliche Analoga erlaubt eine Lagerung der GlucDH unter Stressbedingungen (z.B. erhöhte Temperatur) auch in Gegenwart eines Cofaktors.

Mit einem solchen System lassen sich Blutglucose-Teststreifen mit erheblich verbesserten Stabilitätseigenschaften generieren, bei denen eine Darreichungsform ohne Trockenmittel möglich ist.

### Beispiel 2

Einer Alkoholnachweislösung wurden cNAD oder NAD zugesetzt. Diese Mischungen wurden bei 35 °C eingelagert. Anschließend wurde in regelmäßigen Abständen die Stabilität des Enzyms überprüft und eine Bestimmung der Restaktivität des Enzyms vorgenommen.

Wiederum zeigt eine gelelektrophoretische Analyse im SDS-Gel (Figuren 12 und 13) den Unterschied zwischen einer Lagerung in Anwesenheit von NAD bzw. cNAD. So unterscheiden sich die Banden der mit cNAD stabilisierten Alkohol-Dehydrogenase nach Lagerung über 65 Stunden bei 6 °C bzw. 35 °C nur geringfügig, was für eine Stabilisierung des Enzyms durch das artifizielle Coenzym spricht. Demgegenüber ist die Bande des in Gegenwart von NAD bei 35 °C gelagerten Enzyms vollkommen verschwunden (Figur 12).

Ferner wird aus Figur 13 deutlich, dass die Bande des mit NAD stabilisierten und bei 35 °C gelagerten Enzyms mit zunehmender Lagerungsdauer schwächer wird und nach 5 Tagen nahezu vollständig verschwunden ist. Eine nach 6 Tagen Lagerung bei 35 °C detektierte Bande des mit cNAD stabilisierten Enzyms zeigt eine deutlich geringere Zersetzung des Enzyms und damit eine erhöhte Stabilität.

Dieses Ergebnis läßt sich auch bei Lagerung in flüssiger Phase bestätigen. (Figur 14). Nach 65 Stunden bei 35 °C liegt die Restaktivität von Alkohol-Dehydrogenase in Gegenwart des nativen Coenzyms NAD bei etwa 6 %, während die Restaktivität des Enzyms in Gegenwart des artifiziellen Coenzyms cNAD noch bei etwa 60 % liegt.

### Beispiel 3

Zur Bestimmung von Glucose wurden verschiedene Testsysteme, welche jeweils Glucose-Dehydrogenase, NAD, einen Mediator und ggf. einen optischen Indikator umfassten, photometetrisch bzw. elektrochemisch vermessen.

Für photometetrische Messungen wurden zunächst vier Testelemente, welche jeweils 11 Wochen bei Raumtemperatur gelagert worden waren und neben Glucose-Dehydrogenase, NAD und einem Mediator 2,18-Phosphormolybdänsäure enthielten, bei verschiedenen Glucose-Konzentrationen untersucht.

Wie aus Figur 15 ersichtlich ist, wurde bei allen vier eingesetzten Mediatoren, d.h. [(4-Nitrosophenyl)imino]dimethanol-Hydrochlorid (Med A), 1-Methyl-5,6-dioxo-5,6-dihydro-1,10-phenanthrolinium-trifluormethansulfonat (Med B), 7-Methyl-5,6-dioxo-5,6-dihydro-1,7-phenanthrolinium-trifluormethansulfonat (Med F) und 1-(3-Carboxypropoxy)-5-ethylphenazinium-trifluormethansulfonat (Med G), mit steigender Glucose-Konzentration ein Abfall der Remission beobachtet, womit oben genannte Mediatoren grundsätzlich für eine Bestimmung von Glucose mittels Photometrie geeignet sind.

Bei hohen Glucose-Konzentrationen im Bereich von 800 mg/dl beträgt die Remission der vermessenen Probe bei Verwendung von [(4-Nitrosophenyl)imino]dimethanol-Hydrochlorid bzw. 1-(3-Carboxypropoxy)-5-ethylphenazinium-trifluormethansulfonat noch etwa 20%, was auf die besondere Eignung dieser beiden Mediatoren für photometrische Messungen unter Verwendung des Systems Glucose-Dehydrogenase/NAD, und damit auch des Systems Glucose-Dehydrogenase/cNAD, schließen lässt. Die Kinetik der Umsetzung von Glucose mittels des Systems Glucose-Dehydrogenase, NAD, 1-(3-Carboxypropoxy)-5-ethylphenazinium-trifluormethansulfonat und 2,18-Phosphormolybdänsäure bei Glucose-Konzentrationen im Bereich von 0 bis 800 mg/dl ist in Figur 16 dargestellt.

Wie der schematischen Darstellung in Figur 17 zu entnehmen ist, kann die photometrische Bestimmung von Glucose auch unter (zusätzlicher) Verwendung von Diaphorase als Zwischenmediator erfolgen. Figur 18 zeigt für das System Glucose-Dehydrogenase, NAD, Diaphorase, [(4-Nitrosophenyl)imino]dimethanol-Hydrochlorid und 2,18-Phosphormolybdänsäure (System 1) einen konzentrationsabhängigen Abfall der Remission. Als Vergleich diente das System Glucose-Dye-Oxidoreduktase, Pyrrolochinolinchinon, [(4-Nitrosophenyl)imino]dimethanol-Hydrochlorid und 2,18-Phosphormolybdänsäure (System 2), welches gleichermaßen einen konzentrationsabhängigen Abfall der Remission bedingt, aufgrund der geringen Spezifität von Glucose-Dye-Oxidoreduktase jedoch Nachteile besitzt. Die Kinetik der Umsetzung von Glucose mittels des Systems 1 bei Glucose-Konzentrationen im Bereich von 0 bis 800 mg/dl ist in Figur 19 abgebildet.

Alternativ zur Photometrie kann zum Zwecke der Analytbestimmung auch eine elektrochemische Messung eingesetzt werden. So zeigte sich sowohl für ein Testelement, welches neben Glucose-Dehydrogenase NAD als Coenzym und 1-(3-Carboxypropoxy)-5-ethylphenazinium-trifluormethansulfonat als Mediator enthielt, als auch für ein korrespondierendes System, welches anstelle von NAD das stabilisierte Coenzym cNAD umfasste, eine lineare Abhängigkeit des zur Reoxidation des reduzierten Mediators benötigten Stromes von der Glucose-Konzentration (Figur 20).

Damit ist gezeigt, dass die Analytbestimmung unter Verwendung des Systems Dehydrogenase/stabiles Coenzym auch mittels elektrochemischer Detektion und Auswertung mit einer anderen Wellenlänge, welche unabhäbgig vom Coenzym ist, erfolgen kann. Durch den Einsatz des stabilen Enzym/Coenzym-Paares sollte die Gesamtrezeptur auch weitergehend stabilisiert werden.

## Patentansprüche

1. Verfahren zur Stabilisierung eines Enzyms,
**dadurch gekennzeichnet,**
**dass** man das Enzym für eine Dauer von mindestens 2 Wochen in Gegenwart eines stabilen Coenzyms lagert, wobei das stabile Coenzym aus der Verbindung der Formel (I) 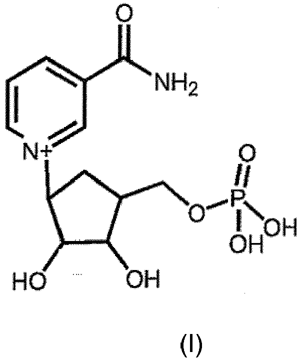 und Verbindungen mit der allgemeinen Formel (II) ausgewählt wird: 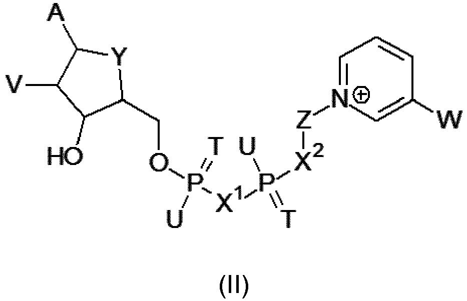 mit
A = Adenin oder ein Analog davon,
T = jeweils unabhängig O, S,
U = jeweils unabhängig OH, SH, BH₃⁻, BCNH₂⁻,
V = jeweils unabhängig OH oder eine Phosphatgruppe, oder zwei Gruppen, die eine cyclische Phosphatgruppe bilden;
W = COOR, CON(R)₂, COR, CSN(R)₂ mit R = jeweils unabhängig H oder C₁-C₂-Alkyl,
X¹, X² = jeweils unabhängig O, CH₂, CHCH₃, C(CH₃)₂, NH, NCH₃,
Y = NH, S, O, CH₂,
Z = ein linearer oder cyclischer organischer Rest ist, mit der Maßgabe, dass Z und der Pyridin-Rest nicht durch eine glycosidische Verbindung verknüpft sind, oder ein Salz oder gegebenenfalls eine reduzierte Form davon.

2. Verfahren nach Anspruch 1,
**dadurch gekennzeichnet,**
**dass** das Enzym aus Dehydrogenasen ausgewählt wird.

3. Verfahren nach Anspruch 2,
**dadurch gekennzeichnet,**
**dass** das Enzym eine Dehydrogenase ist, ausgewählt aus einer Glucose-Dehydrogenase (E.C.1.1.1.47), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), alpha-Hydroxybutyrat-Dehydrogenase, Sorbitol-Dehydrogenase oder einer Aminosäure-Dehydrogenase, wie etwa L-Aminosäure-Dehydrogenase (E.C.1.4.1.5).

4. Verfahren nach einem der Ansprüche 1-3,
**dadurch gekennzeichnet,**
**dass** als Enzym eine Glucose-Dehydrogenase verwendet wird.

5. Verfahren nach einem der Ansprüche 1-4, worin das stabile Coenzym aus Verbindungen der allgemeinen Formel (II) ausgewählt ist und Z ausgewählt wird aus
(i)einem linearen Rest mit 4-6 C-Atomen, worin 1 oder 2 Atome gegebenenfalls durch ein oder mehrere Heteroatome ausgewählt aus O, S und N ersetzt sind, und
(ii) einem Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

6. Verfahren nach Anspruch 5, worin Z eine Verbindung der allgemeinen Formel (III) 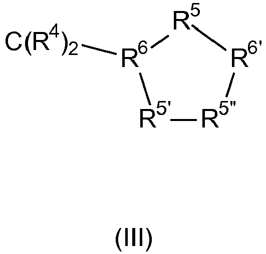 ist,
wobei zwischen R⁵' und R⁵" eine Einfach- oder Doppelbindung vorliegen kann, mit
R⁴ = jeweils unabhängig H, F, Cl, CH₃,
R⁵ = CR⁴₂,
wobei R⁵' = O, S, NH, NC₁-C₂-Alkyl, CR⁴₂, CHOH, CHOCH₃, und
R⁵" = CR⁴₂, CHOH, CHOCH₃, wenn zwischen R⁵' und R⁵" eine Einfachbindung vorliegt,
wobei R⁵'=R⁵"=CR⁴, wenn zwischen R⁵' und R⁵" eine Doppelbindung vorliegt, und
R⁶, R⁶' = jeweils unabhängig CH oder CCH₃.

7. Verfahren nach einem der Ansprüche 1-6, worin das stabile Coenzym aus Verbindungen der allgemeinen Formel (II) ausgewählt ist mit W = CONH₂ oder COCH₃.

8. Verfahren nach Anspruch 6 oder 7, worin R⁵ CH₂ ist.

9. Verfahren nach einem der Ansprüche 6-8, worin R⁵' ausgewählt ist aus CH₂, CHOH und NH.

10. Verfahren nach einem der Ansprüche 6-9, worin R⁵' und R⁵" jeweils CHOH sind.

11. Verfahren nach einem der Ansprüche 6-9, worin R⁵' NH und R⁵" CH₂ ist.

12. Verfahren nach einem der Ansprüche 1-11,
**dadurch gekennzeichnet,**
**dass** das stabile Coenzym carbaNAD ist.

13. Verfahren nach einem der Ansprüche 1-12,
**dadurch gekennzeichnet,**
**dass** man das mit einem stabilen Coenzym stabilisierte Enzym bei einer Temperatur von mindestens 20°C lagert.

14. Verfahren nach einem der Ansprüche 1-13,
**dadurch gekennzeichnet,**
**dass** man das mit einem stabilen Coenzym stabilisierte Enzym ohne Trocknungsreagenz lagert.

15. Verfahren nach einem der Ansprüche 1-14,
**dadurch gekennzeichnet,**
**dass** man das mit einem stabilen Coenzym stabilisierte Enzym bei einer relativen Luftfeuchtigkeit von mindestens 50 % lagert.

16. Verfahren nach einem der Ansprüche 1-15,
**dadurch gekennzeichnet,**
**dass** die Lagerung des mit einem stabilen Coenzym stabilisierten Enzyms als Trockensubstanz erfolgt.

17. Verfahren nach einem der Ansprüche 1-16,
**dadurch gekennzeichnet,**
**dass** die Lagerung des mit einem stabilen Coenzym stabilisierten Enzyms in flüssiger Phase erfolgt.

18. Verfahren nach einem der Ansprüche 1-17,
**dadurch gekennzeichnet,**
**dass** die Lagerung des mit einem stabilen Coenzym stabilisierten Enzyms auf einem Testelement erfolgt.

19. Enzym, welches mit einem stabilen Coenzym stabilisiert ist,
**dadurch gekennzeichnet,**
**dass** es bei einer Lagerung von vorzugsweise mindestens 2 Wochen bei einer Temperatur von vorzugsweise mindestens 20°C, gegebenenfalls bei hoher Luftfeuchtigkeit und ohne Trockenreagenz, eine Abnahme der enzymatischen Aktivität von weniger als 50 % gegenüber dem Ausgangswert aufweist, wobei das Enzym eine mutierte Glucose-Dehydrogenase ist, die ausgewählt ist aus Gluc-DH E96G_E170K gemäß SEQ ID No. 2 und GlucDH E170K_K252L gemäß SEQ ID No. 1 und wobei das stabile Coenzym aus der Verbindung der Formel (I) 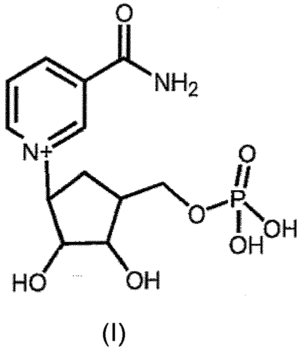 und Verbindungen mit der allgemeinen Formel (II) ausgewählt wird: 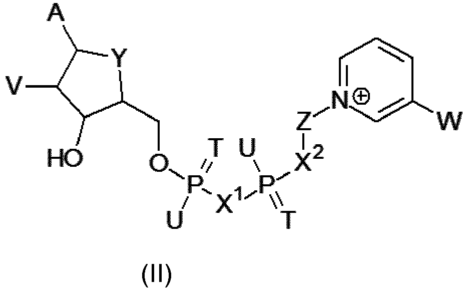 mit
A = Adenin oder ein Analog davon,
T = jeweils unabhängig O, S,
U = jeweils unabhängig OH, SH, BH3-, BCNH2-,
V = jeweils unabhängig OH oder eine Phosphatgruppe, oder zwei Gruppen, die eine cyclische Phosphatgruppe bilden;
W = COOR, CON(R)2, COR, CSN(R)2 mit R = jeweils unabhängig H oder C1-C2-Alkyl,
X1, X2 = jeweils unabhängig O, CH2, CHCH3, C(CH3)2, NH, NCH3,
Y = NH, S, O, CH2,
Z = ein linearer oder cyclischer organischer Rest ist, mit der Maßgabe, dass Z und der Pyridin-Rest nicht durch eine glycosidische Verbindung verknüpft sind, oder ein Salz oder gegebenenfalls eine reduzierte Form davon.

20. Nachweisreagenz zur Bestimmung eines Analyten, welches ein stabilisiertes Enzym nach Anspruch 19 enthält.

21. Testelement,
**dadurch gekennzeichnet,**
**dass** es ein stabilisiertes Enzym nach Anspruch 19 oder ein Nachweisreagenz nach Anspruch 20 enthält.

## Claims

1. Method for stabilising an enzyme,
**characterised in that**
the enzyme is stored for a period of at least 2 weeks in the presence of a stable coenzyme, in which the stable coenzyme is selected from the compound of formula (I) 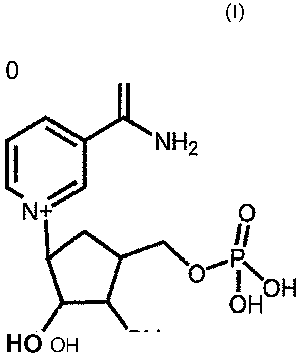 and compounds with general formula (II): 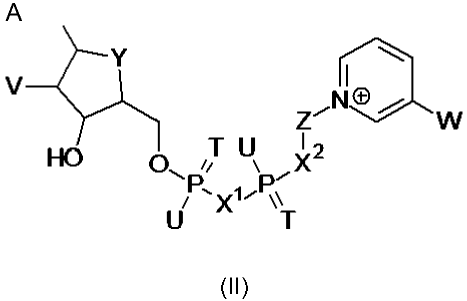 with
A = adenine or an analogue thereof,
T = each independently O, S,
U = each independently OH, SH, BH₃', BCNH₂',
V = each independently OH or a phosphate group or two groups that form a cyclic phosphate group;
W = COOR, CON(R)₂, COR, CSN(R)₂ with R = each independently H or C₁-C₂ alkyl,
X¹, X² = each independently O, CH₂, CHCH₃, C(CH₃)₂, NH, NCH₃,
Y = NH, S, O, CH₂,
Z = a linear or cyclic organic residue, providing that Z and the pyridine residue are not linked by a glycosidic compound or a salt or if necessary a reduced form thereof.

2. Method according to claim 1,
**characterised in that**
the enzyme is selected from dehydrogenases.

3. Method according to claim 2,
**characterised in that**
the enzyme is a dehydrogenase, selected from a glucose dehydrogenase (E.C.1.1.1.47), lactate dehydrogenase (E.C.1.1.1.27, 1.1.1.28), malate dehydrogenase (E.C.1.1.1.37), glycerol dehydrogenase (E.C.1.1.1.6), alcohol dehydrogenase (E.C.1.1.1.1), alpha hydroxybutyrate dehydrogenase, sorbitol dehydrogenase or an amino acid dehydrogenase, such as L-amino-acid dehydrogenase (E.C.1.4.1.5).

4. Method according to one of claims 1-3,
**characterised in that**
a glucose dehydrogenase is used as enzyme.

5. Method according to one of claims 1-4, in which the stable coenzyme is selected from compounds of general formula (II) and Z is selected from
(i) a linear residue with 4-6 C atoms, in which 1 or 2 atoms are replaced if necessary by one or several heteroatoms selected from O, S and N, and
(ii) a residue comprising a cyclic group with 5 or 6 C atoms, which if necessary contains a heteroatom selected from O, S and N as well as if necessary one or several substituents, and a CR⁴₂ residue, in which CR⁴₂ is bound to the cyclic group and to X², with R⁴ = each independently H, F, Cl, CH₃.

6. Method according to claim 5, in which Z is a compound of general formula (III), 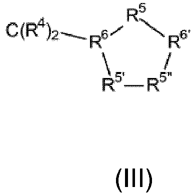 is,
in which there may be a single or double bond between R⁵' und R⁵", with
R⁴ = each independently H, F, Cl, CH₃,
R⁵ = CR⁴₂,
in which R⁵' = O, S, NH, NC₁-C₂ alkyl, CR⁴₂, CHOH, CHOCH₃, and R⁵" = CR⁴₂, CHOH, CHOCHs, if there is a single bond between R⁵' and R⁵",
in which R⁵' = R⁵" = CR⁴, if there is a double bond between R⁵' and R⁵", and R⁶, R⁶' = each independently CH or CCH₃

7. Method according to one of claims 1-6, in which the stable coenzyme is selected from compounds of general formula (II) with W = CONH₂ or COCH₃.

8. Method according to claim 6 or 7, in which R⁵ is CH₂.

9. Method according to one of claims 6-8, in which R⁵' is selected from CH₂, CHOH and NH.

10. Method according to one of claims 6-9, in which R⁵' and R⁵" are each CHOH.

11. Method according to one of claims 6-9, in which R⁵' is NH and R⁵" is CH₂.

12. Method according to one of claims 1-11,
**characterised in that**
the stable coenzyme is carba-NAD.

13. Method according to one of claims 1-12,
**characterised in that**
the enzyme stabilised with a stable coenzyme is stored at a temperature of at least 20°C.

14. Method according to one of claims 1-13,
**characterised in that**
the enzyme stabilised with a stable coenzyme is stored without drying agent.

15. Method according to one of claims 1-14,
**characterised in that**
the enzyme stabilised with a stable coenzyme is stored at a relative humidity of at least 50%.

16. Method according to one of claims 1-15,
**characterised in that**
the enzyme stabilised with a stable coenzyme is stored as a dry substance.

17. Method according to one of claims 1-16,
**characterised in that**
the enzyme stabilised with a stable coenzyme is stored in the liquid phase.

18. Method according to one of claims 1-17,
**characterised in that**
the enzyme stabilised with a stable coenzyme is stored on a test element.

19. Enzyme, which is stabilised with a stable coenzyme,
**characterised in that**
with storage of preferably at least 2 weeks at a temperature of preferably at least 20° C, if necessary at high humidity and without drying agent, it has a reduction in enzymatic activity of less than 50% compared with the initial value, in which the enzyme is a mutated glucose dehydrogenase, which is selected from Gluc-DH E96G_E170K according to SEQ ID No. 2 and GlucDH E170K_K252L according to SEQ ID No. 1 and in which the stable coenzyme is selected from the compound of formula (I) 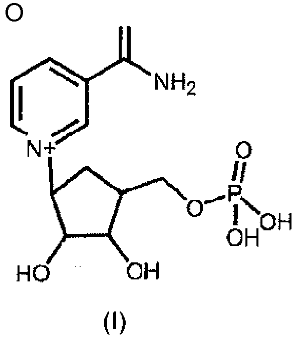 and compounds with general formula (II): 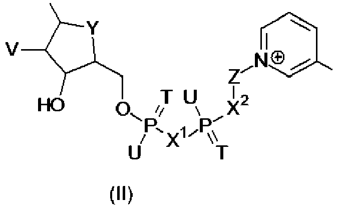 with
A = adenine or an analogue thereof,
T = each independently O, S,
U = each independently OFI, SH, BH3-, BCNFI2-,
V = each independently OFI or a phosphate group or two groups that form a cyclic phosphate group;
W = COOR, CON(R)2, COR, CSN(R)2 with R = each independently H or C1-C2 alkyl,
X1, X2 = each independently O, CH2, CHCH3, C(CH3)2, NH, NCH3,
Y = NH, S, O, CH2,
Z = a linear or cyclic organic residue,
providing that Z and the pyridine residue are not linked by a glycosidic compound or a salt or if necessary a reduced form thereof.

20. Detection reagent for determining an analyte, which contains a stabilised enzyme according to claim 19.

21. Test element,
**characterised in that**
it contains a stabilised enzyme according to claim 19 or a detection reagent according to claim 20.

## Revendications

1. Procédé de stabilisation d'une enzyme,
**caractérisé en ce que**
ladite enzyme est stockée pour une durée d'au moins 2 semaines en présence d'une coenzyme stable, ladite coenzyme stable étant choisie parmi le composé de formule (I) 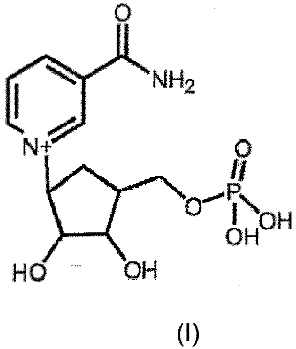 et les composés répondant à la formule générale (II): 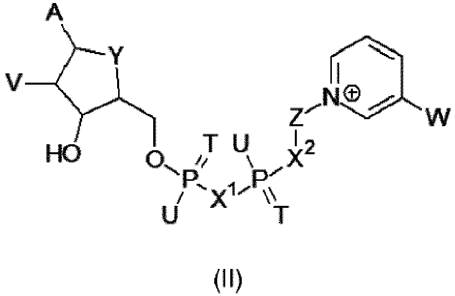 avec
A = adénine ou un analogue de celle-ci,
T = O, S, de manière indépendante des autres,
U = OH, SH, BH₃⁻, BCNH₂⁻, de manière indépendante des autres,
V = OH, ou un groupe phosphate, ou deux groupes formant un groupe phosphate cyclique, de manière indépendante des autres ;
W = COOR, CON(R)₂, COR, CSN(R)₂ avec R = H ou C₁-C₂-Alkyl, de manière indépendante des autres,
X¹, X² = O, CH₂, CHCH₃, C(CH₃)₂, NH, NCH₃, de manière indépendante des autres,
Y = NH, S, O, CH₂,
Z = un radical organique linéaire ou cyclique, à condition que Z et le radical de pyridine ne soient pas liés par une liaison glycosidique, ou un sel de celui-ci ou, éventuellement, une forme de celui-ci ayant subi une réduction.

2. Procédé selon la revendication 1,
**caractérisé en ce que**
ladite enzyme est choisie parmi les déshydrogénases.

3. Procédé selon la revendication 2,
**caractérisé en ce que**
ladite enzyme est une déshydrogénase choisie parmi une glucose déshydrogénase (E.C.1.1.1.47), lactate déshydrogénase (E.C.1.1.1.27, 1.1.1.28), malate déshydrogénase (E.C.1.1.1.37), glycérol déshydrogénase (E.C.1.1.1.6), alcool déshydrogénase (E.C.1.1.1.1), alpha-hydroxybutyrate déshydrogénase, sorbitol déshydrogénase ou une aminoacide déshydrogénase telle qu'une L-aminoacide déshydrogénase (E.C.1.4.1.5).

4. Procédé selon l'une des revendications 1 à 3,
**caractérisé en ce que**
l'on utilise une glucose déshydrogénase en tant qu'enzyme.

5. Procédé selon l'une des revendications 1 à 4, ladite coenzyme stable étant choisie parmi les composés répondant à la formule générale (II) et Z étant choisi parmi
(i) un radical linéaire renfermant 4 à 6 atomes de carbone, dans lequel 1 ou 2 atomes sont éventuellement remplacés par un ou plusieurs hétéroatomes choisis parmi O, S et N, et
(ii) un radical qui comprend un groupe cyclique renfermant 5 ou 6 atomes de C et contenant éventuellement un hétéroatome choisi parmi O, S et N, et éventuellement un ou plusieurs substituants, et un radical CR⁴₂, CR⁴₂ étant lié audit groupe cyclique et à X², avec R⁴ = H, F, Cl, CH₃, de manière indépendante des autres.

6. Procédé selon la revendication 5, Z étant un composé répondant à la formule générale (III) 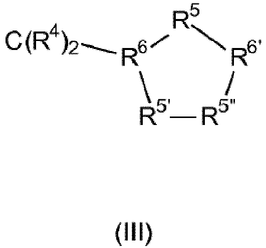
R⁵' et R⁵" pouvant être reliés par une liaison simple ou double, avec
R⁴ = H, F, Cl, CH₃, de manière indépendante des autres,
R⁵ = CR⁴₂,
R⁵' étant = O, S, NH, NC₁-C₂-alkyle, CR⁴₂, CHOH, CHOCH₃, et
R⁵" étant = CR⁴₂, CHOH, CHOCH₃, si R⁵' et R⁵" sont reliés par une liaison simple,
R⁵' étant = R⁵" étant = CR⁴, si R⁵' et R⁵" sont reliés par une liaison double, et
R⁶, R⁶' = CH ou CCH₃, de manière indépendante des autres.

7. Procédé selon l'une des revendications 1 à 6, ladite coenzyme stable étant choisie parmi les composés répondant à la formule générale (II), avec W = CONH₂ ou COCH₃.

8. Procédé selon les revendications 6 ou 7, R⁵ représentant CH₂.

9. Procédé selon l'une des revendications 6 à 8, R⁵' étant choisi parmi CH₂, CHOH et NH.

10. Procédé selon l'une des revendications 6 à 9, R⁵' et R⁵" représentant chacun CHOH.

11. Procédé selon l'une des revendications 6 à 9, R⁵' représentant NH et R⁵" représentant CH₂.

12. Procédé selon l'une des revendications 1 à 11,
**caractérisé en ce que**
ladite coenzyme stable est de la carbaNAD.

13. Procédé selon l'une des revendications 1 à 12,
**caractérisé en ce que**
ladite enzyme, stabilisée au moyen d'une coenzyme stable, est stockée à une température supérieure ou égale à 20 °C.

14. Procédé selon l'une des revendications 1 à 13,
**caractérisé en ce que**
ladite enzyme, stabilisée au moyen d'une coenzyme stable, est stockée sans réactif de déshydratation.

15. Procédé selon l'une des revendications 1 à 14,
**caractérisé en ce que**
ladite enzyme, stabilisée au moyen d'une coenzyme stable, est stockée à un taux d'humidité supérieur ou égal à 50 %.

16. Procédé selon l'une des revendications 1 à 15,
**caractérisé en ce que**
ladite enzyme, stabilisée au moyen d'une coenzyme stable, est stockée sous forme de substances sèches.

17. Procédé selon l'une des revendications 1 à 16,
**caractérisé en ce que**
ladite enzyme, stabilisée au moyen d'une coenzyme stable, est stockée en phase liquide.

18. Procédé selon l'une des revendications 1 à 17,
**caractérisé en ce que**
ladite enzyme, stabilisée au moyen d'une coenzyme stable, est stockée sur un élément d'essai.

19. Enzyme, stabilisée au moyen d'une coenzyme stable,
**caractérisée en ce que**
elle subit une perte d'activité enzymatique inférieure à 50 %, par rapport à la valeur de départ, lorsqu'on la stocke préférentiellement durant au moins 2 semaines à une température préférentiellement supérieure ou égale à 20 °C, éventuellement à un taux d'humidité élevé et sans réactif de déshydratation, ladite enzyme étant une glucose déshydrogénase ayant subi une mutation, choisie parmi la Gluc-DH E96G_E170K selon la SEQ ID n° 2 et la GlucDH E170K_K252L selon la SEQ ID n° 1, et ladite coenzyme stable étant choisie parmi le composé de formule (I) 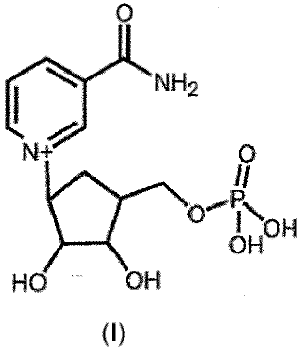 et les composés répondant à la formule générale (II) : 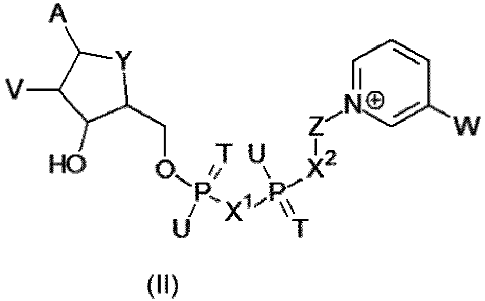 avec
A = adénine ou un analogue de celle-ci,
T = O, S, de manière indépendante des autres,
U = OH, SH, BH3-, BCNH2-, de manière indépendante des autres,
V = OH, ou un groupe phosphate, ou deux groupes formant un groupe phosphate cyclique, de manière indépendante des autres ;
W = COOR, CON(R)2, COR, CSN(R)2 avec R = H ou C1-C2-Alkyl, de manière indépendante des autres,
X1, X2 = O, CH2, CHCH3, C(CH3)2, NH, NCH3, de manière indépendante des autres,
Y = NH, S, O, CH2,
Z = un radical organique linéaire ou cyclique, à condition que Z et le radical de pyridine ne soient pas liés par une liaison glycosidique, ou un sel de celui-ci ou, éventuellement, une forme de celui-ci ayant subi une réduction.

20. Réactif de détection qui est destiné à réaliser le dosage d'un analyte et qui contient une enzyme stabilisée selon la revendication 19.

21. Elément d'essai
**caractérisé en ce qu'il**
contient une enzyme stabilisée selon la revendication 19, ou un réactif de détection selon la revendication 20.


### SEQUENCE LISTING

<110> F. Hoffmann - La Roche
<120> Stabilisierung von Dehydrogenasen mit stabilen Coenzymen
<130> 40444P WO
<150> EP 08 003 054.7
   <151> 2008-02-19
<160> 2
<170> PatentIn version 3.3
<210> 1
   <211> 261
   <212> PRT
   <213> Artificial
<220>
   <223> Mutant of glucose dehydrogenase from Bacillus subtilis
<220>
   <221> MUTAGEN
   <222> (96)..(96)
<220>
   <221> MUTAGEN
   <222> (170)..(170)
<400> 1
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
           35                  40                  45
   Lys Glu Glu Val Ile Lys Ala Gly Gly Glu Ala Val Val Val Gln Gly
       50                  55                  60
   Asp Val Thr Lys Glu Glu Asp Val Lys Asn Ile Val Gln Thr Ala Ile
   65                  70                  75                  80
   Lys Glu Phe Gly Thr Leu Asp Ile Met Ile Asn Asn Ala Gly Leu Gly
                   85                  90                  95
   Asn Pro Val Pro Ser His Glu Met Pro Leu Lys Asp Trp Asp Lys Val
               100                 105                 110
   Ile Gly Thr Asn Leu Thr Gly Ala Phe Leu Gly Ser Arg Glu Ala Ile
           115                 120                 125
   Lys Tyr Phe Val Glu Asn Asp Ile Lys Gly Asn Val Ile Asn Met Ser
       130                 135                 140
   Ser Val His Glu Val Ile Pro Trp Pro Leu Phe Val His Tyr Ala Ala
   145                 150                 155                 160
   Ser Lys Gly Gly Ile Lys Leu Met Thr Lys Thr Leu Ala Leu Glu Tyr
                   165                 170                 175
   Ala Pro Lys Gly Ile Arg Val Asn Asn Ile Gly Pro Gly Ala Ile Asn
               180                 185                 190
   Thr Pro Ile Asn Ala Glu Lys Phe Ala Asp Pro Lys Gln Lys Ala Asp
           195                 200                 205
   Val Glu Ser Met Ile Pro Met Gly Tyr Ile Gly Glu Pro Glu Glu Ile
       210                 215                 220
   Ala Ala Val Ala Val Trp Leu Ala Ser Lys Glu Ser Ser Tyr Val Thr
   225                 230                 235                 240
   Gly Ile Thr Leu Phe Ala Asp Gly Gly Met Thr Lys Tyr Pro Ser Phe
                   245                 250                 255
   Gln Ala Gly Arg Gly
               260
<210> 2
   <211> 261
   <212> PRT
   <213> Artificial
<220>
   <223> Mutant of glucose dehydrogenase from Bacillus subtilis
<220>
   <221> MUTAGEN
   <222> (170)..(170)
<220>
   <221> MUTAGEN
   <222> (252)..(252)
<400> 2
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
           35                  40                  45
   Lys Glu Glu Val Ile Lys Ala Gly Gly Glu Ala Val Val Val Gln Gly
       50                  55                  60
   Asp Val Thr Lys Glu Glu Asp Val Lys Asn Ile Val Gln Thr Ala Ile
   65 70 75 80
   Lys Glu Phe Gly Thr Leu Asp Ile Met Ile Asn Asn Ala Gly Leu Glu
   85 90 95
   Asn Pro Val Pro Ser His Glu Met Pro Leu Lys Asp Trp Asp Lys Val
   100 105 110
   Ile Gly Thr Asn Leu Thr Gly Ala Phe Leu Gly Ser Arg Glu Ala Ile
   115 120 125
   Lys Tyr Phe Val Glu Asn Asp Ile Lys Gly Asn Val Ile Asn Met Ser
   130 135 140
   Ser Val His Glu Val Ile Pro Trp Pro Leu Phe Val His Tyr Ala Ala
   145 150 155 160
   Ser Lys Gly Gly Ile Lys Leu Met Thr Lys Thr Leu Ala Leu Glu Tyr
   165 170 175
   Ala Pro Lys Gly Ile Arg Val Asn Asn Ile Gly Pro Gly Ala Ile Asn
   180 185 190
   Thr Pro Ile Asn Ala Glu Lys Phe Ala Asp Pro Lys Gln Lys Ala Asp
   195 200 205
   Val Glu Ser Met Ile Pro Met Gly Tyr Ile Gly Glu Pro Glu Glu Ile
   210 215 220
   Ala Ala Val Ala Val Trp Leu Ala Ser Lys Glu Ser Ser Tyr Val Thr
   225 230 235 240
   Gly Ile Thr Leu Phe Ala Asp Gly Gly Met Thr Leu Tyr Pro Ser Phe
   245 250 255
   Gln Ala Gly Arg Gly
   260